# FASCICULE DE BREVET EUROPEEN

(11) **EP 1 915 975 B1**
(45) Date de publication et mention de la délivrance du brevet: **04.04.2012**
(21) Numéro de dépôt: 07356153.2
(22) Date de dépôt: 25.10.2007
(51) Int. Cl.: A61F 2/42

(54) **Prothèse de cheville avec réglage de position neutre**
Sprunggelenkprothese mit Regulierung der Neutralstellung
Ankle prosthesis with neutral position adjustment

(30) Priorité: 26.10.2006 FR 0609417
(43) Date de publication de la demande: 30.04.2008
(73) Titulaire: Newdeal, 69800 Saint-Priest (FR)
(72) Inventeur: Hintermann, Beat, 4410 Liestal (CH)
(74) Mandataire: Martin, Didier Roland Valéry

(56) Documents cités:
- WO-A-91/07931
- US-A1- 2005 288 792

## Description

La présente invention se rapporte au domaine technique des prothèses de cheville destinées à permettre le traitement orthopédique de l'articulation de la cheville, et plus particulièrement aux implants visant à restituer l'anatomie de l'articulation de la cheville.

La présente invention concerne une prothèse de cheville comprenant un implant talien destiné à être implanté dans ou sur le talus, un implant tibial destiné à être implanté dans ou sur le tibia, ainsi qu'un implant intermédiaire destiné à être interposé entre ledit implant tibial et ledit implant talien, ledit implant intermédiaire étant en outre conçu pour être monté mobile relativement audit implant talien au niveau d'une interface de contact, afin d'autoriser une mobilité de la cheville.

La présente invention concerne également un procédé de fabrication en usine d'une prothèse de cheville telle que décrite ci-dessus.

La présente invention concerne également une prothèse de cheville d'essai.

La présente invention concerne également un kit chirurgical destiné à la mise en place d'une prothèse de cheville.

Il est connu d'avoir recours à des prothèses de cheville afin de restaurer une certaine liberté de mouvement de l'articulation de la cheville après que cette dernière a été endommagée totalement ou partiellement, par exemple suite à un choc traumatique ou une maladie.

Il est en particulier connu d'implanter des prothèses de cheville comprenant trois implants, à savoir un implant talien destiné à être implanté dans ou sur le talus (astragale), un implant tibial destiné à être implanté dans ou sur le tibia, ainsi qu'un implant intermédiaire destiné à être interposé entre ledit implant tibial et ledit implant talien.

Généralement, l'implant intermédiaire possède une importante liberté de mouvement entre les deux autres implants. Plus particulièrement, il repose généralement en appui-plan sur l'implant tibial de manière à autoriser des mouvements de translation antéro-postérieure, de translation médio-latérale et de rotation selon l'axe médullaire du tibia sensiblement perpendiculaire à la surface dudit appui-plan.

Usuellement, l'interface de contact entre l'implant intermédiaire et l'implant talien est constituée d'une surface de frottement bombée, généralement de forme cylindrique, sphérique ou tronconique, afin d'autoriser la flexion plantaire et dorsale du pied par rapport à la jambe.

On connaît également par le document WO-91/07931 (VOLVO) une prothèse de cheville conforme au préambule de la revendication 1. Si elles offrent des résultats intéressants sur le plan cinématique, de telles prothèses de cheville peuvent toutefois se révéler inadaptées à la condition du patient traité.

En effet, lorsque le patient a déjà fait l'objet d'un traitement orthopédique qui limitait la mobilité de sa cheville, l'articulation présente généralement une instabilité due à une fragilisation par atrophie musculaire et/ou à une laxité des tendons. Ainsi, lorsqu'il est par exemple envisagé de réaliser une arthroplastie destinée à restaurer la mobilité articulaire alors que l'articulation avait été antérieurement immobilisée par arthrodèse, ou encore lorsque l'on souhaite procéder au remplacement d'une prothèse d'un modèle ancien dont la configuration cinématique diffère de la prothèse à trois implants, la restauration brutale de nombreux degrés de liberté auparavant supprimés tend à perturber l'équilibre du patient et expose ce dernier à des risques de chutes, de blessures et de détérioration des tissus situés au niveau de l'articulation concernée.

Dès lors, la mise en oeuvre de prothèses à trois implants de l'art antérieur implique une très longue rééducation de tels patients.

Par ailleurs, certaines exigences réglementaires nationales interdisent l'emploi des prothèses à trois implants de l'art antérieur.

Les objets assignés à l'invention visent par conséquent à proposer une nouvelle prothèse de cheville ne présentant pas les inconvénients susmentionnés et qui, tout en offrant une mobilité articulaire satisfaisante, garantit une bonne stabilité à l'articulation de la cheville.

Un autre objet de l'invention vise à proposer une nouvelle prothèse de cheville qui présente une bonne ergonomie et un bon confort d'utilisation pour le patient.

Un autre objet de l'invention vise à proposer une nouvelle prothèse de cheville au sein de laquelle les phénomènes d'usure sont minimisés et qui présente une longévité accrue.

Un autre objet de l'invention vise à proposer une nouvelle prothèse de cheville qui soit de conception particulièrement simple et robuste.

Un autre objet de l'invention vise à proposer une nouvelle prothèse de cheville dont la mise en oeuvre, et notamment l'implantation, est particulièrement facile.

Un autre objet de l'invention vise à proposer une nouvelle méthode de préparation d'une prothèse de cheville qui confère à ladite prothèse de cheville une bonne stabilité.

Un autre objet de l'invention vise à proposer une nouvelle méthode de préparation d'une prothèse de cheville qui confère à ladite prothèse de cheville une bonne ergonomie.

Un autre objet de l'invention vise à proposer une nouvelle prothèse de cheville d'essai qui simplifie et fiabilise la mise en oeuvre d'une prothèse de cheville conforme à l'invention.

Un autre objet de l'invention vise à proposer une nouvelle prothèse de cheville d'essai qui soit de conception simple et peu onéreuse.

Un autre objet de l'invention vise à proposer un nouveau kit chirurgical destiné à la mise en place d'une prothèse de cheville qui, tout en offrant une mobilité articulaire satisfaisante, garantit une bonne stabilité à l'articulation de la cheville, ledit kit permettant une implantation simple, précise et fiable de ladite prothèse de cheville.

Un autre objet de l'invention vise à proposer un nouveau kit chirurgical destiné à la mise en place d'une prothèse de cheville dont la conception soit particulièrement simple et la mise en oeuvre particulièrement facile.

Un autre objet de l'invention vise à proposer une nouvelle méthode chirurgicale d'implantation d'une prothèse de cheville qui permette de conférer à l'articulation une bonne stabilité et une mobilité satisfaisante.

Un autre objet de la présente invention vise à proposer une nouvelle méthode chirurgicale d'implantation de prothèse de cheville qui optimise la longévité de la prothèse et améliore son confort d'utilisation par le patient.

Enfin, un autre objet de l'invention vise à proposer une nouvelle méthode chirurgicale d'implantation d'une prothèse de cheville qui soit particulièrement simple à mettre en oeuvre, précise, fiable et reproductible.

Les objets assignés à l'invention sont atteints à l'aide d'une prothèse de cheville définie dans la revendication 1.

Les objets assignés à l'invention sont également atteints à l'aide d'un procédé de fabrication en usine d'une prothèse de cheville défini dans la revendication 11.

Les objets assignés à l'invention sont également atteints à l'aide d'une prothèse de cheville d'essai définie dans la revendication 13.

Les objets assignés à l'invention sont également atteints à l'aide d'un kit chirurgical destiné à la mise en place d'une prothèse de cheville défini dans la revendication 20.

D'autres caractéristiques et avantages de l'invention apparaîtront plus en détails à la lecture de la description qui suit, ainsi qu'à l'aide des dessins annexés fournis à titre purement illustratif et non limitatif, parmi lesquels :
- La figure 1 illustre, selon une vue en perspective, une partie d'une variante de réalisation d'une prothèse de cheville conforme à l'invention.
- La figure 2 illustre, selon une vue en perspective, une prothèse de cheville conforme à l'invention au sein de laquelle est mise en oeuvre la partie représentée sur la figure 1.
- La figure 3 illustre, selon une vue en perspective, la prothèse de cheville illustrée sur la figure 2 implantée au niveau de l'articulation de la cheville.
- La figure 4 illustre, selon une vue en perspective, la mise en oeuvre d'une première partie d'un kit chirurgical conforme à l'invention.
- La figure 5 illustre, selon une vue en perspective, la mise en oeuvre d'une seconde partie d'un kit chirurgical conforme à l'invention.
- La figure 6 illustre, selon une vue en perspective, une variante de réalisation d'implant tibial et de moyen d'accouplement au sein d'une prothèse de cheville conforme à l'invention.
- La figure 7 illustre, selon une vue en perspective, une autre variante de réalisation d'implant tibial et de moyen d'accouplement au sein d'une prothèse de cheville conforme à l'invention.

La prothèse de cheville 1 conforme à l'invention est destinée à restaurer au moins partiellement la mobilité de l'articulation de la cheville chez un patient victime notamment d'une maladie ou d'un choc traumatique.

Elle peut également être utilisée pour remplacer une prothèse de cheville précédemment implantée.

La prothèse de cheville 1 conforme à l'invention comprend un implant talien 2 destiné à être implanté dans ou sur le talus 3 (astragale), ainsi qu'un implant tibial 4 destiné à être implanté dans ou sur le tibia 5.

La prothèse de cheville 1 comprend également un implant intermédiaire 6 qui est destiné à être interposé entre l'implant tibial 4 et l'implant talien 2.

Ledit implant intermédiaire 6 est conçu pour être monté mobile relativement audit implant talien 2 au niveau d'une interface de contact 7, afin d'autoriser une mobilité de la cheville. Il peut notamment être constitué de polyéthylène.

Plus précisément, l'implant intermédiaire 6 présente de préférence une surface de contact 6A destinée à venir en appui contre une surface 2A de l'implant talien 2 de forme conjuguée, de telle sorte que l'implant intermédiaire puisse se déplacer par glissement, avec frottement, relativement à l'implant talien 2.

De façon particulièrement préférentielle, les surfaces de contact conjuguées 2A, 6A présentent une forme bombée, par exemple sensiblement sphérique, cylindrique ou tronconique, de manière à autoriser les mouvements de flexion plantaire et de flexion dorsale du pied par rapport à la jambe.

Selon une caractéristique importante de l'invention, la prothèse 1 est pourvue d'un moyen d'accouplement configurable 10 conçu pour permettre l'agencement de l'implant intermédiaire 6 par rapport à l'implant tibial 4, selon une configuration spécifique choisie parmi une pluralité de configurations possibles.

En d'autres termes, le moyen d'accouplement 10 permet d'intervenir sur la prothèse 1 de manière à permettre au praticien de sélectionner au cas par cas, parmi plusieurs choix potentiels, l'agencement de l'implant intermédiaire 6 par rapport à l'implant tibial 4 qui lui paraît le plus approprié.

Ainsi, le moyen d'accouplement 10 est embarqué et conçu d'une part pour autoriser le réglage de la position de l'implant intermédiaire 6 par rapport à l'implant tibial 4 afin de permettre à la prothèse 1 d'adopter intrinsèquement plusieurs configurations fonctionnelles possibles, et d'autre part pour permettre la sélection de l'agencement de l'implant intermédiaire 6 par rapport à l'implant tibial 4 selon une configuration spécifique choisie parmi cette pluralité de configurations fonctionnelles possibles.

Par « *configuration fonctionnelle* », on entend à la fois la disposition spatiale statique (position et orientation des différents éléments constitutifs de la prothèse) et l'agencement cinématique (degrés de liberté) qui seront mis en oeuvre dans le cadre du fonctionnement normal de la prothèse après implantation définitive de celle-ci, afin de permettre la restauration appropriée chez le patient concerné du ou des degrés de liberté articulaires souhaités.

Par « *embarqué* », on indique que, au sens de l'invention, le moyen d'accouplement 10 est incorporé dans sa globalité à la prothèse et possède intrinsèquement une variabilité d'agencement qui autorise sa reconfiguration propre, et, partant, la modification de la configuration fonctionnelle de la prothèse. Avantageusement, cette possibilité de réglage autonome par un moyen unique configurable en lui-même par construction permet d'éviter le gaspillage de temps et de matière première susceptible d'être provoqué par l'utilisation systématique de lots d'implants à usage unique interchangeables, de forme et de dimensions diverses, destinés à être implantés alternativement jusqu'à l'obtention d'une configuration satisfaisante.

Au sens de l'invention, le moyen d'accouplement configurable 10 conforme à l'invention permet d'améliorer la stabilité de la prothèse 1, et par conséquent celle de l'articulation de la cheville, en plaçant sous contrôle la mobilité relative de l'implant intermédiaire 6 et de l'implant tibial 4, par exemple en la bornant.

Selon l'invention, il est envisageable que le moyen d'accouplement 10 soit destiné à simplement restreindre l'amplitude d'un ou plusieurs débattements autorisés par la liaison cinématique qu'il réalise entre l'implant intermédiaire 6 et l'implant tibial 4. Par exemple, si l'implant intermédiaire 6 se trouve en appui-plan contre l'implant tibial 4, comme c'est le cas dans les prothèses à trois implants de l'art antérieur, le moyen d'accouplement 10 conforme à l'invention peut comprendre des butées ou des éléments de guidage limitant les courses en translation dans le plan de contact, par exemple de manière à ce que la liaison ne puisse pas être totalement déboîtée par un mouvement incontrôlé de trop forte amplitude.

Plus particulièrement le moyen d'accouplement 10 permet de choisir une position nominale « *de base* » qu'occupe l'implant intermédiaire 6 par rapport à l'implant tibial 4 et de limiter le nombre de degrés de liberté et/ou l'amplitude des mouvements possibles dudit implant intermédiaire 6 par rapport audit implant tibial 4 autour de cette position nominale. En d'autres termes, le moyen d'accouplement 10 permet alors de confiner le débattement relatif autour d'une position de référence déterminée, librement choisie parmi une pluralité de positions possibles autorisant chacune le fonctionnement de la prothèse.

De façon particulièrement préférentielle, le moyen d'accouplement 10 est destiné à réaliser une liaison cinématique de type encastrement entre l'implant intermédiaire 6 et l'implant tibial 4, c'est-à-dire à supprimer tout degré de liberté entre lesdits implants. A cet effet, ledit moyen d'accouplement configurable 10 comporte de préférence des moyens d'encastrement 11 dudit implant intermédiaire 6 sur ledit implant tibial 4.

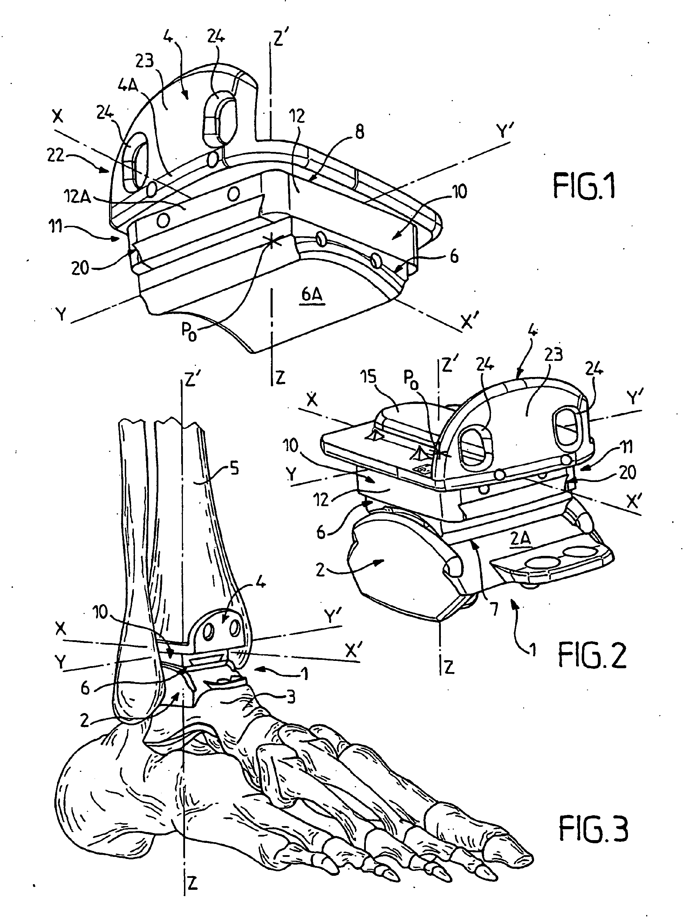

Ainsi, selon une variante de réalisation préférentielle illustrée notamment sur les figures 1 et 2, le moyen d'accouplement 10 constitue un moyen de fixation conçu pour assujettir l'implant intermédiaire 6 à l'implant tibial 4.

En d'autres termes, le moyen d'accouplement 10 permet de préférence de bloquer l'implant intermédiaire 6 contre l'implant tibial 4 dans une position de référence sélectionnée à l'issue d'un réglage préliminaire autorisé par ce même moyen d'accouplement.

Dans la mesure où le moyen d'accouplement 10 conforme à l'invention permet de brider, voire de supprimer, les mouvements de l'implant intermédiaire 6 par rapport à l'implant tibial 4, des contraintes mécaniques apparaissent au niveau de ces implants lorsque la prothèse 1 est sollicitée dans le cadre des mouvements effectués par le patient, notamment lors de la marche.

De telles contraintes sont susceptibles d'occasionner une usure prématurée de l'un ou l'autre des implants 2, 4, 6 constitutifs de la prothèse 1, voire de créer une déformation ou même une rupture desdits implants 2, 4, 6.

C'est pourquoi, la configuration spécifique est de préférence choisie en fonction de la conformation articulaire particulière du patient sur lequel doit être implantée la prothèse 1. En d'autres termes, il est avantageusement possible d'effectuer un ajustement de la prothèse 1 conforme à l'invention en fonction de l'anatomie propre du patient.

A ce titre, il est remarquable que moyen d'accouplement 10 conforme à l'invention est prévu pour rendre accessibles au moment de l'implantation, par construction, plusieurs configurations fonctionnelles de la prothèse qui assurent chacune la restauration de la mobilité de l'articulation du pied par rapport à la jambe.

Or, parmi cette pluralité de configurations fonctionnelles que la prothèse 1 peut adopter par construction lors de l'implantation, on comprend que, pour un patient donné, la configuration fonctionnelle qui se rapprochera le plus d'une reproduction fidèle de l'articulation naturelle dudit patient présentera un mode de fonctionnement « *optimisé* », tandis que les autres configurations fonctionnelles, plus éloignées de l'anatomie du patient, auraient tendance à contraindre l'articulation naturelle et ainsi offrir un mode de fonctionnement relativement « *dégradé* », c'est-à-dire moins performant que le mode « *optimisé* » en termes de confort pour le patient et d'usure de la prothèse.

De façon particulièrement préférentielle, la configuration spécifique de l'implant intermédiaire 6 par rapport à l'implant tibial 4 est donc choisie de telle sorte que les contraintes exercées sur l'implant intermédiaire 6 et l'implant tibial 4 lors des mouvements naturels de la cheville soient globalement minimisées. Elle est alors dite « *configuration neutre* ».

Plus précisément, il convient de rappeler que chez un patient sain, l'articulation de la cheville autorise plusieurs mouvements naturels du tibia 5 par rapport au talus 3, et notamment, en sus des mouvements de flexion dorsale et plantaire :
- une translation antéro-postérieure orientée sensiblement selon un axe (XX'), lorsque la jambe tend à « *avancer* » ou « *reculer* » par rapport au pied tandis que ledit pied reste fixe,
- une translation médio-latérale orientée sensiblement selon un axe (YY') lorsque la jambe tend à se déplacer latéralement en rentrant vers l'entrejambe du patient ou en fuyant vers l'extérieur tandis que ledit pied reste fixe,
- une rotation selon l'axe médullaire (ZZ') du tibia, lorsque la jambe tend à pivoter selon un angle de lacet par rapport au pied qui reste fixe.

Les trois mouvements décrits ci-dessus sont généralement de faible amplitude.

Ainsi que cela est illustré sur la figure 3, lorsque le patient se tient droit en station debout et que son pied repose sur un sol plat et horizontal, l'axe (XX') est sensiblement horizontal et parallèle au plan sagittal du patient (c'est-à-dire orienté selon la direction à laquelle fait face le patient), l'axe (YY') est sensiblement horizontal, orienté selon un abord latéral au patient, et sensiblement orthogonal à l'axe (XX'), tandis que l'axe (ZZ') s'étend sensiblement selon la verticale et forme une normale au plan défini par les axes (XX') et (YY').

Or, il est connu qu'il existe pour chaque patient, en fonction de son anatomie propre, un point d'origine P₀ autour duquel s'effectuent les débattements en translation antéro-postérieure, en translation médio-latérale et en rotation autour de l'axe médullaire (ZZ') du tibia 5. Géométriquement, le point d'origine P₀ correspond sensiblement à l'intersection virtuelle des axes (XX'), (YY') et (ZZ').

De plus, dans le cas où une prothèse 1 est implantée chez le patient, celle-ci matérialise par construction un ou plusieurs axes cinématiques qui permettent de restaurer, au moins partiellement, un ou plusieurs des degrés de liberté de l'articulation.

La configuration neutre au sens de l'invention correspond donc préférentiellement à une position de l'implant intermédiaire 6 relativement à l'implant tibial 4 dans laquelle ledit implant intermédiaire 6 est sensiblement centré par rapport au point d'origine P₀ et dans laquelle l'implant tibial 4 et/ou l'implant intermédiaire 6 sont orientés de telle sorte que les axes cinématiques de la prothèse 1 coïncident sensiblement avec les axes anatomiques naturels de la cheville du patient.

A titre d'exemple, si la zone de contact 8 entre l'implant intermédiaire 6 et l'implant tibial 4 est agencée de manière à former un appui-plan, il conviendra que ladite zone de contact 8 soit sensiblement normale à l'axe (ZZ') et localisée au voisinage proche des axes de translation (XX') et (YY') et de préférence superposée au plan formé par ces derniers.

Dans ce qui suit, on considèrera par commodité de description que l'expression « *configuration neutre* » s'applique indifféremment à la prothèse 1 dans son ensemble, au moyen d'accouplement 10, ou à tout autre élément constitutif de la prothèse 1 lorsque celui-ci est considéré dans la situation qu'il occupe lorsque l'agencement de l'implant intermédiaire 6 par rapport à l'implant tibial 4 correspond à la configuration neutre.

De façon particulièrement avantageuse, en offrant la possibilité de placer l'implant intermédiaire 6 en configuration neutre, le moyen d'accouplement configurable 10 conforme à l'invention permet de minimiser l'amplitude d'éventuels déplacements résiduels dudit implant intermédiaire 6 par rapport audit implant tibial 4 et/ou de limiter les contraintes exercées sur ces éléments lorsque lesdits implants 4, 6 sont solidarisés l'un à l'autre.

De surcroît, cette possibilité de réglage offerte par le moyen d'accouplement 10 permet avantageusement d'adapter la prothèse 1 selon la conformation articulaire propre à chaque patient, ce qui permet un ajustement fin, précis et personnalisé de ladite prothèse 1.

On peut ainsi prévenir dans une large mesure l'inconfort d'utilisation et l'usure excessive qui résulteraient inévitablement d'un bridage arbitraire de l'implant intermédiaire 6 sur l'implant tibial 4 selon une configuration unique pré-déterminée par construction au niveau de la prothèse elle-même et qui ne correspondrait pas systématiquement à l'anatomie du patient concerné.

Selon une variante de réalisation préférentielle illustrée notamment sur les figures 1, 2, 5, 6 et 7, le moyen d'accouplement configurable 10 comporte une embase 12 conçue pour être fixée sur l'implant tibial 4 et former une interface mécanique entre ledit implant tibial 4 et l'implant intermédiaire 6.

Ladite embase 12 se présentera de préférence sensiblement sous la forme générale d'un parallélépipède rectangle.

De préférence, ladite embase 12 dispose de moyens de fixation réglables 14 qui permettent de choisir, parmi une pluralité de positions possibles, la position dans laquelle ladite embase 12 est effectivement fixée par rapport à l'implant tibial 4.

Bien entendu, la configuration de la zone de contact 8 entre l'implant tibial 4 et l'embase 12 n'est pas limitée à une forme particulière de réalisation. Toutefois, de façon préférentielle, la zone de contact entre l'implant tibial 4 et l'embase 12 sera sensiblement plane et, de façon particulièrement préférentielle, coïncidera sensiblement avec le plan défini par les axes (XX') et (YY').

Ainsi, il sera avantageusement possible de plaquer l'embase 12 contre l'implant tibial 4 et de la positionner par rapport à ce dernier selon une grande variété de positions et d'orientations, en utilisant les degrés de liberté offerts par la liaison appui-plan libre ainsi créée. La fixation définitive destinée à bloquer fermement l'embase 12 peut intervenir ensuite, après que la configuration neutre a été repérée.

Selon une variante de réalisation préférentielle, les moyens de fixation réglables 14 permettent un réglage continu de la position de fixation de l'embase 12, par rapport à l'implant tibial 4, par exemple à l'aide d'un couple vis-trou oblong ou de tout autre mécanisme approprié.

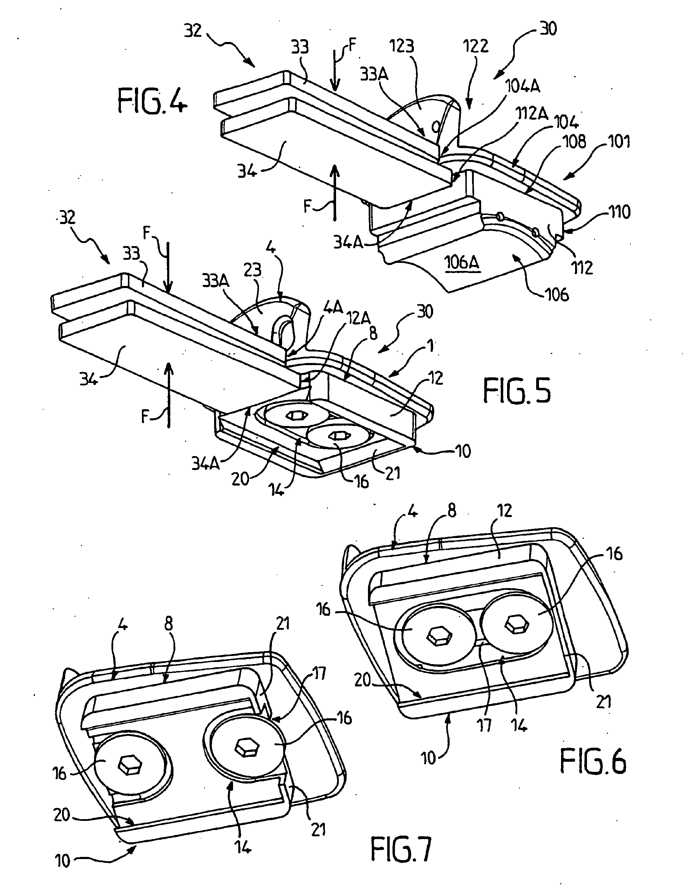

En particulier, tel que cela est représenté sur les figures 5 à 7, les moyens de fixation réglables 14 pourront comporter une pluralité de vis à tête large 16 coopérant avec une ou plusieurs rainures 17 ménagées dans l'embase 12 et dimensionnées de telle sorte que le jeu résiduel entre les tiges des vis 16 et les bords de rainure 17 permettent un ajustement linéaire et/ou angulaire de la position de ladite embase 12 par rapport à l'implant tibial 4. Ainsi, il sera possible de lier dans un premier temps de façon mobile l'embase 12 à l'implant tibial 4, puis d'ajuster sa position et enfin de serrer les vis 16 afin de plaquer et d'immobiliser ladite embase 12 contre l'implant tibial 4. Un bossage 15, tel que celui représenté sur la figure 2, pourra en outre être prévu sur l'implant tibial 4 d'une part pour fournir un élément de repérage permettant un positionnement médio-latéral reproductible dudit implant tibial 4 par rapport au tibia 5, et d'autre part pour conférer audit implant tibial 4 l'épaisseur de matière requise par l'utilisation des vis 16.

En d'autres termes, les moyens de fixation réglable 14, et plus globalement le moyen d'accouplement configurable 10, sont conçus pour adopter alternativement d'une part un état « *déverrouillé* » dans lequel ils autorisent une mobilité relative des éléments constitutifs de la prothèse, et notamment de l'implant intermédiaire 6 et/ou de l'embase 12 par rapport à l'implant tibial 4, ladite mobilité étant adaptée et suffisante pour permettre l'évolution de la prothèse entre plusieurs configurations fonctionnelles possibles, et plus particulièrement pour permettre le positionnement de l'implant intermédiaire *6 (via* l'embase 12) par rapport audit implant tibial 4 selon plusieurs agencements spatiaux distincts, et d'autre part un état « *verrouillé* » dans lequel ils restreignent ladite mobilité, et de préférence immobilisent au moins deux éléments constitutifs de la prothèse l'un par rapport à l'autre, préférentiellement l'implant intermédiaire 6 (via l'embase 12) par rapport à l'implant tibial 4, dans une, ou au voisinage d'une, position particulière librement choisie parmi la pluralité de positions possibles qui correspondent aux différentes configurations fonctionnelles de la prothèse.

Selon une variante de réalisation préférentielle, l'embase 12 est pourvue de moyens d'accueil 20 permettant le raccordement de l'implant intermédiaire 6 à ladite embase 12.

De façon particulièrement préférentielle, lesdits moyens d'accueil 20 sont agencés de telle sorte que l'implant intermédiaire 6 ne puisse occuper qu'une position unique et prédéterminée par rapport à l'embase 12.

Plusieurs solutions sont envisageables pour réaliser de tels moyens d'accueil 20, et notamment des systèmes à rainure et languette ou encore des systèmes à vis, de préférence associés à des détrompeurs tels que des ergots, entailles ou plots de centrage.

Selon une variante de réalisation préférentielle illustrée notamment sur les figures 1, 2 et 5, les moyens d'accueil 20 seront formés par une queue d'aronde, l'implant intermédiaire 6 pouvant alors être inséré par glissement latéral dans l'embase 12.

Selon cette variante, il suffit ensuite de bloquer l'implant intermédiaire en translation selon l'axe générateur de la queue d'aronde pour obtenir une liaison encastrement entre ladite embase 12 et ledit implant intermédiaire 6.

De préférence, on prévoira à cet effet un élément de butée 21 contre lequel l'implant intermédiaire 6 vient en appui lorsqu'il est inséré à fond dans l'embase 12, ainsi qu'un moyen anti-retour (non représenté) qui s'oppose à l'extraction de l'implant intermédiaire 6 hors de l'embase 12 après que celui-ci a été inséré en butée dans ladite embase.

De façon bien connue de l'homme du métier, un tel moyen anti-retour pourra par exemple être formé par un encliquetage, en particulier à l'aide d'une lèvre mobile faisant saillie sur l'une des faces de l'implant intermédiaire 6 qui rentrent au contact de l'embase 12, ladite lèvre se déformant élastiquement ou s'escamotant lors de l'insertion de l'implant intermédiaire 6 dans l'embase 12 pour se redéployer ensuite dans une encoche prévue à cet effet dans ladite embase lorsque ledit implant intermédiaire a atteint sa position de butée.

Ainsi, selon l'invention, les moyens d'encastrement 11 de l'implant intermédiaire 6 sur l'implant tibial 4 peuvent avantageusement être formés par la combinaison de l'embase 12, de ses moyens de fixation réglables 14 et de ses moyens d'accueil 20.

Il est par ailleurs remarquable qu'une fois en position dans la queue d'aronde, l'implant intermédiaire 6 peut avantageusement servir à bloquer les têtes des vis de fixation 16 et empêcher ainsi toute apparition inopinée de jeu entre l'embase 12 et l'implant tibial 4 par desserrage accidentel desdites vis 16.

Bien entendu, le moyen d'accouplement 10 configurable conforme à l'invention n'est nullement limité à une variante particulière de réalisation et peut notamment inclure d'autres moyens de réglage embarqués permettant de configurer les divers implants constitutifs de la prothèse les uns par rapport aux autres.

Selon une variante de réalisation préférentielle, la prothèse de cheville 1 comprend un moyen de repérage 22 conçu pour indiquer l'agencement de l'implant intermédiaire 6 par rapport à l'implant tibial 4 afin de permettre au praticien de configurer précisément le moyen d'accouplement configurable 10. En d'autres termes, ledit moyen de repérage 22 renseigne le praticien sur la configuration dans laquelle se trouve le moyen d'accouplement configurable 10, et plus généralement la prothèse 1.

Plus particulièrement, le moyen de repérage 22 conforme à l'invention peut constituer un moyen de repérage spatial conçu pour renseigner le praticien sur la disposition de l'implant intermédiaire 6 et/ou de l'embase 12 par rapport à l'implant tibial 4.

Ainsi, il est avantageusement possible de connaître et par conséquent, tel que cela sera détaillé ci-dessous, de reproduire l'agencement de la prothèse 1 qui correspond à une configuration particulière, notamment à la configuration neutre.

De préférence, le moyen de repérage 22 comprend une première surface de repérage 4A associée à l'implant tibial 4 et une seconde surface de repérage 12A associée à l'embase 12, la position relative desdites première et seconde surfaces de repérage 4A, 12A permettant ainsi de déterminer, et plus précisément de matérialiser visuellement et physiquement, la position de ladite embase 12 dans le repère que forme l'implant tibial 4.

Selon une variante de réalisation préférentielle, l'implant tibial 4 présente une languette 23 qui forme à la fois un bouclier tibial empêchant un développement inconsidéré de cellules osseuses susceptibles de gêner le fonctionnement de l'articulation et une patte de fixation pourvue de trous oblongs 24 destinés à recevoir des vis de fixation osseuse. La première surface de repérage 4A peut alors avantageusement être formée par un secteur de la face antérieure de la languette 23, de préférence sensiblement plan.

De manière analogue, la seconde surface de repérage 12A sera de préférence formée par une face sensiblement plane de l'embase 12.

Avantageusement, tel que cela sera détaillé ci-après, la première et la seconde surface de repérage 4A, 12A permettent de réaliser un réglage précis de la position de l'embase 12 par rapport à l'implant tibial 4 lors de l'assemblage de la prothèse 1.

La présente invention concerne également une méthode de préparation d'une prothèse de cheville 1 comprenant un implant talien 2 destiné à être implanté dans ou sur le talus 3, un implant tibial 4 destiné à être implanté dans ou sur le tibia 5 ainsi qu'un implant intermédiaire 6 destiné à être interposé entre ledit implant tibial 4 et ledit implant talien 2, ledit implant intermédiaire 6 étant en outre conçu pour être monté mobile relativement audit implant talien 2 au niveau d'une interface de contact 7 afin d'autoriser une mobilité de la cheville, caractérisée en ce qu'elle comprend une étape (E1) d'agencement au cours de laquelle on agence l'implant intermédiaire 6 par rapport à l'implant tibial 4, à l'aide d'un moyen d'accouplement configurable 10 dont est pourvue la prothèse 1, selon une configuration fonctionnelle spécifique que l'on choisit, au cours d'une étape (E2) de sélection, parmi une pluralité de configurations fonctionnelles possibles.

De préférence, au cours de l'étape (E2) de sélection, on choisit la configuration spécifique en fonction de la conformation articulaire particulière du patient sur lequel doit être implantée la prothèse 1, et, de façon particulièrement préférentielle, on choisit comme configuration spécifique la configuration neutre propre audit patient.

Selon une première variante, ladite méthode de préparation peut avantageusement être mise en oeuvre en usine, en tant qu'étape d'assemblage d'un procédé de fabrication sur-mesure d'une prothèse de cheville devant être livrée pré-assemblée en configuration neutre.

Il est en particulier envisageable qu'un praticien hospitalier, après avoir effectué un diagnostic et des relevés bio-métriques sur son patient, par exemple par imagerie médicale, commande à distance une prothèse 1 au fabricant, en vue d'une intervention à venir, en transmettant simplement à celui-ci un cahier des charges dimensionnel recensant les données-clefs relatives à l'assemblage des éléments constitutifs de la prothèse, et en lui laissant le soin de procéder au montage et au conditionnement appropriés de ladite prothèse.

Selon une autre variante (non revendiquée en tant que telle), ladite méthode de préparation peut être mise en oeuvre au cours d'une intervention chirurgicale par le chirurgien, lorsqu'il configure lui-même la prothèse 1 juste au moment de son implantation.

Bien entendu, l'invention concerne également un procédé de fabrication en usine d'une prothèse 1, ledit procédé comportant une étape de réalisation d'un moyen d'accouplement configurable 10 au cours de laquelle on réalise un moyen d'accouplement configurable 10 embarqué conçu d'une part pour autoriser le réglage de la position dudit implant intermédiaire 6 par rapport audit implant tibial 4 afin de permettre à la prothèse 1 d'adopter intrinsèquement plusieurs configurations fonctionnelles possibles, et d'autre part pour permettre la sélection de l'agencement de l'implant intermédiaire 6 par rapport à l'implant tibial 4 selon une configuration spécifique choisie parmi cette pluralité de configurations fonctionnelles possibles.

La présente invention concerne également une prothèse de cheville d'essai 101 conçue pour être implantée provisoirement en lieu et place d'une prothèse de cheville définitive pouvant être configurée selon une configuration fonctionnelle spécifique choisie parmi une pluralité de configurations fonctionnelles possibles, ladite prothèse de cheville d'essai étant d'une part agencée de manière à pouvoir adopter *in vivo* l'une ou l'autre desdites configurations fonctionnelles possibles et d'autre part pourvue de moyens de repérage 122 agencés pour permettre au praticien de relever la configuration dans laquelle elle se trouve de manière à pouvoir reproduire celle-ci sur la prothèse de cheville définitive.

Au sens de l'invention, la prothèse de cheville d'essai 101 et la prothèse de cheville définitive sont de préférence destinées à être utilisées au cours d'une même intervention chirurgicale unique, la prothèse de cheville d'essai étant d'abord mise en place au sein de l'articulation de la cheville afin de permettre l'identification et le repérage de la configuration neutre propre au patient, puis ensuite retirée et remplacée par la prothèse de cheville définitive 1 dont l'implant intermédiaire 6 sera positionné et fixé à l'implant tibial 4 en fonction du résultat du repérage spatial de la configuration neutre effectué sur la prothèse de cheville d'essai 101.

En d'autres termes, la prothèse de cheville d'essai 101 constitue de préférence un accessoire d'implantation intermédiaire qui permet d'effectuer sur le patient le relevé topographique nécessaire à la bonne configuration de la prothèse de cheville définitive, et non une prothèse fonctionnelle destinée à séjourner durablement dans l'articulation et à supporter les contraintes liées à la marche.

Avantageusement, la prothèse de cheville d'essai 101 pourra donc présenter une structure simplifiée par rapport à la prothèse de cheville définitive et notamment être réalisée à partir de matériaux moins résistants et meilleur marché que ceux employés pour la prothèse de cheville définitive.

De préférence, la prothèse de cheville définitive est formée par une prothèse de cheville 1 telle que décrite dans ce qui précède et la prothèse de cheville d'essai 101 comprend un implant talien d'essai 102, un implant tibial d'essai 104, un implant intermédiaire d'essai 106 et un moyen d'accouplement configurable d'essai 110 dont les formes et les dimensions correspondent sensiblement à celles de l'implant talien 2, de l'implant implant tibial 4, de l'implant intermédiaire 6 et du moyen d'accouplement configurable 10 respectivement.

Par convention de numérotation, les références aux éléments constitutifs de la prothèse de cheville d'essai 101 correspondront aux références, incrémentées d'une valeur 100, des éléments constitutifs analogues de la prothèse de cheville définitive.

Selon une variante de réalisation préférentielle, l'implant intermédiaire d'essai 106 et le moyen d'accouplement configurable d'essai 110, et plus précisément l'embase d'essai 112 de ce dernier, forment un ensemble monobloc de type patin 106, 110, tel que cela est illustré sur la figure 4.

A ce titre, il est remarquable que, selon la présente invention, il n'est pas forcément nécessaire de reproduire exactement à l'identique sur la prothèse de cheville d'essai 101 le moyen d'accouplement configurable 10.

En effet, si l'on opte pour une variante de réalisation de la prothèse de cheville définitive 1 selon laquelle l'implant intermédiaire 6 ne peut occuper qu'une position unique par rapport à l'embase 12, il est nécessaire et suffisant, pour définir complètement la configuration neutre, de connaître uniquement la position de l'un ou l'autre de ces deux éléments par rapport à l'implant tibial 4.

D'autre part, dans la mesure où seule la mobilité de l'implant intermédiaire d'essai 106 par rapport à l'implant tibial d'essai 104 est alors intéressante pour rechercher la position adéquate que l'on souhaite conférer à l'embase 12, il n'est pas indispensable de prévoir l'équivalent des moyens de fixation 14 sur la prothèse de cheville d'essai 101.

On peut donc avantageusement modéliser au sein de la prothèse de cheville d'essai 101 cet ensemble formé par l'embase 12 et l'implant intermédiaire 6, assimilable à un solide unique sur le plan cinématique, par un patin 106, 110 reproduisant uniquement les dimensions et les formes extérieures utiles dudit ensemble.

Selon une variante de réalisation particulièrement préférentielle, la surface du patin 106, 110 destinée à entrer en contact avec l'implant tibial d'essai 104, au niveau de la zone de contact 108, est sensiblement plane, lisse, et exempte de moyens de fixation.

Bien entendu, d'autres simplifications des éléments constitutifs de la prothèse de cheville d'essai 101 peuvent intervenir par rapport à leurs homologues de la prothèse de cheville définitive 1 sans sortir du cadre de la présente invention, pourvu que lesdites simplifications ne soient pas préjudiciables à la faisabilité et à la validité de la détermination de la position neutre.

De telles simplifications, associées à l'emploi de matériaux bon marché, permettent avantageusement de produire à moindre coût une prothèse de cheville d'essai susceptible d'être stérilisée et utilisée à plusieurs reprises.

De plus, la prothèse de cheville d'essai 101 conforme à l'invention peut avantageusement être pourvue de moyens d'auto-centrage agencés pour amener ladite prothèse de cheville d'essai dans une configuration neutre, dans laquelle les contraintes exercées sur l'implant intermédiaire d'essai 106 et l'implant tibial d'essai 104 lors des mouvements naturels de la cheville soient globalement minimisées. En d'autres termes, lesdits moyens d'auto-centrage tendent spontanément à placer ladite prothèse de cheville d'essai 101 en configuration neutre, de préférence sous l'effet de mouvements naturels que le chirurgien imprime au pied du patient par rapport à la jambe de ce dernier.

A cet effet, les moyens d'auto-centrage sont de préférence formés par le patin 106, 110 lui-même, qui est alors agencé pour être interposé librement entre l'implant talien d'essai 102 et l'implant tibial d'essai 104 de manière à se trouver d'une part en liaison appui-plan avec ce dernier, au niveau de l'interface de contact 108, et plus particulièrement dans le plan défini par les axes (XX') et (YY'), et d'autre part en liaison pivot, de préférence pivot glissant, avec l'implant talien d'essai 102, au niveau de sa surface bombée 106A.

Un tel agencement confère au patin 106, 110 un comportement auto-centreur du fait que, lorsque la prothèse de cheville d'essai 101 est implantée et que le patin 106, 110 se trouve positionné entre l'implant tibial d'essai 104 et l'implant talien d'essai 102, au contact avec ces derniers au niveau de ses faces opposées, une manipulation par le chirurgien du pied par rapport à la jambe peut entraîner, sous l'effet des contraintes dynamiques mises en oeuvre au sein de l'articulation, un déplacement progressif dudit patin 106, 110 selon les degrés de liberté autorisé par l'appui-plan, jusqu'à ce que ledit patin atteigne une position sensiblement centrée sur la position d'origine P₀ dans laquelle l'état des contraintes dynamiques est globalement minimisé.

De préférence, les moyens de repérage 122 sont agencés pour coopérer avec les organes tactiles 33, 34 d'un moyen de mesure 32 conçu pour permettre au praticien de relever la configuration de la prothèse de cheville d'essai 101 alors que cette dernière se trouve implantée au sein du patient.

De façon particulièrement préférentielle, lesdits moyens de repérage 122 sont agencés pour être solidarisés avec lesdits organes tactiles 33, 34 avant implantation de ladite prothèse de cheville d'essai de manière à pouvoir coopérer avec ces derniers pendant que la prothèse de cheville d'essai est implantée. Ainsi, on pourra notamment obtenir un suivi continu des évolutions de la configuration dans laquelle se trouve la prothèse de cheville d'essai *in vivo.*

A ce titre, il est remarquable que le mimétisme dimensionnel et fonctionnel existant entre la prothèse de cheville d'essai 101 et la prothèse de cheville définitive 1 permet avantageusement au praticien d'effectuer un relevé de position direct sur ladite prothèse de cheville d'essai 101, les distances et les orientations des éléments constitutifs de cette dernière traduisant fidèlement la configuration qu'il convient de reproduire avec la prothèse de cheville définitive 1.

La présente invention concerne également un kit chirurgical 30 destiné à la mise en place d'une prothèse de cheville.

Selon l'invention, ledit kit chirurgical 30 comporte une prothèse de cheville 1 conforme à l'invention, dite « *prothèse de cheville définitive* », ainsi qu'une prothèse de cheville d'essai 101 telle que décrite précédemment.

De préférence, le kit chirurgical 30 conforme à l'invention comprend en outre un moyen de mesure 32 conçu pour permettre au praticien de relever la configuration de la prothèse de cheville d'essai 101 alors que cette dernière se trouve implantée au sein du patient.

Plus précisément, ledit moyen de mesure 32 est de préférence conçu pour relever la position du moyen d'accouplement d'essai 110, respectivement du patin 106, 110, par rapport à l'implant tibial d'essai 104, notamment lorsque la prothèse de cheville d'essai 101 se trouve en configuration neutre de telle sorte que les contraintes exercées sur l'implant intermédiaire 106 et l'implant tibial 104 lors des mouvements naturels de la cheville soient globalement minimisées.

Le moyen de mesure 32 conforme à l'invention peut indifféremment exploiter une technologie de mesure au contact, de type palpeur, ou à distance, de type faisceau laser. Il peut également recourir à une mesure explicite, par exemple en permettant au praticien de quantifier un décalage linéaire (millimètres) et/ou angulaire (degrés) du patin 106, 110 par rapport à l'implant tibial 104, ou encore, de façon préférentielle, à une mesure implicite de la configuration neutre par conformation d'un gabarit qui matérialise la position relative de ces éléments.

De préférence, le moyen de mesure 32 comporte un premier organe tactile 33 présentant une première surface de palpation 33A et un second organe tactile 34 présentant une seconde surface de palpation 34A destinées à venir respectivement au contact de l'implant tibial d'essai 104 et de l'implant intermédiaire d'essai 106.

Plus précisément, tel que cela est illustré sur la figure 4, la seconde surface de palpation 34A est de préférence agencée pour venir au contact de l'embase d'essai 112 qui forme la base du patin 110.

Selon une variante de réalisation préférentielle, le premier et le second organe tactile 33, 34 sont pourvus de moyens d'association respectivement à l'implant tibial d'essai 104 et à l'implant intermédiaire d'essai 106 de manière à pouvoir d'une part être solidarisés à ces derniers avant l'implantation de la prothèse de cheville d'essai 101 et d'autre part rester solidaires de ces derniers pendant que ladite prothèse de cheville d'essai 101 se trouve dans le patient.

En particulier, on pourra utiliser à cette fin un système de tenons faisant saillie sur les surfaces de palpation et destinés à venir s'ajuster dans des trous ménagés sur une première et une seconde surface de repérage d'essai 104A, 112A associées respectivement à l'implant tibial d'essai 104 et à l'embase 112 du patin 106, 110.

Ainsi, le moyen de mesure 32 conforme à l'invention sera susceptible d'évaluer en permanence la configuration de la prothèse de cheville d'essai 101 implantée en suivant en temps réel les mouvements de l'implant intermédiaire d'essai 106 par rapport à l'implant tibial d'essai 104.

De préférence, les moyens d'association seront agencés pour permettre l'alignement et la fixation des organes tactiles 33, 34 dans une position unique et reproductible avec la surface de repérage d'essai 104A, 112A qui leur est associée.

De façon préférentielle, le premier et le second organe tactile sont formés respectivement par une première et une seconde rallonge conçues pour présenter chacune, lorsque la prothèse de cheville d'essai 101 est implantée, une extrémité qui dépasse hors de l'incision pratiquée pour accéder à la cheville du patient.

De façon particulièrement préférentielle, tel que cela est illustré sur les figures 4 et 5, le premier et le second organe tactile 33, 34 seront formés chacun par une plaque parallélépipédique, les deux plaques pouvant avantageusement être accolées l'une à l'autre pour former une liaison appui-plan libre sensiblement coplanaire à la zone de contact 8, 108, c'est-à-dire au plan formé par les axes (XX') et (YY') lorsque la prothèse de cheville d'essai 101 est implantée.

De préférence, le premier et le second organe tactile 33, 34 sont liés par un moyen de blocage réversible ou amovible de manière à ce qu'ils puissent être laissés mobiles l'un par rapport à l'autre ou au contraire solidarisés l'un à l'autre.

Selon une variante de réalisation particulièrement préférentielle illustrée sur les figures 4 et 5, l'immobilisation relative des deux organes tactiles 33, 34 peut intervenir de façon simple et sûre à l'aide d'une pince-étau (non représentée) qui tend à les comprimer l'un contre l'autre, au niveau de la portion qui fait saillie hors de l'incision, en exerçant un effort de pincement F sensiblement normal au plan de contact 108.

Ainsi, la mise en oeuvre du moyen de blocage réversible ne perturbera pas la position du premier et du second organe tactile 33, 34, ni celle de l'implant tibial d'essai 104 ou du patin 106, 110 une fois la configuration neutre atteinte par la prothèse de cheville d'essai 101.

De préférence, la première et la seconde surface de repérage d'essai 104, 112 sont sensiblement identiques aux première et seconde surfaces de repérage 4, 12 de la prothèse de cheville définitive 1 respectivement, de sorte que le premier et le second organe tactile 33, 34 puissent épouser indifféremment et de manière similaire l'implant tibial 4 définitif ou l'implant tibial d'essai 104, respectivement l'embase définitive 12 ou l'embase d'essai 112.

De plus, la prothèse de cheville définitive 1 présente de préférence des éléments conjugués aux moyens d'association à l'implant tibial d'essai 104 et à l'implant intermédiaire d'essai 106, notamment des trous destinés à accueillir les tenons des organes tactiles 33, 34.

Ainsi, il existe une interchangeabilité entre la prothèse de cheville d'essai 101 et la prothèse de cheville définitive 1, de telle sorte qu'après avoir préalablement repéré la configuration neutre sur la prothèse de cheville d'essai, fixé les organes tactiles 33, 34 l'un à l'autre et déboîté lesdits organes tactiles de la prothèse de cheville d'essai 101, il est possible d'accoler l'implant tibial 4 et l'embase 12 aux première et seconde surfaces de palpation 33A, 34A et ainsi de positionner ces éléments selon la configuration neutre.

En d'autres termes, le moyen de mesure 32 conforme à l'invention est de préférence également conçu pour permettre le report de la configuration de la prothèse de cheville d'essai 101 sur la prothèse de cheville définitive 1, ledit moyen de mesure 32 formant un gabarit d'assemblage de ladite prothèse de cheville définitive 1.

Il est remarquable qu'une telle variante de réalisation préférée du moyen de mesure 32 permet un report rapide, simple et précis de ladite configuration, la prise de mesure sur la prothèse de cheville d'essai 101 et sa restitution sur la prothèse de cheville définitive 1 intervenant immédiatement et intuitivement sans qu'il soit nécessaire au praticien de chercher à connaître explicitement une quelconque valeur intermédiaire, telle qu'un décalage linéaire ou angulaire.

Bien entendu, la présente invention peut concerner le moyen de mesure 32 considéré séparément en tant que tel, et plus précisément un gabarit ajustable permettant de relever physiquement une configuration particulière adoptée par la prothèse de cheville d'essai 101 afin de reporter cette même configuration sur la prothèse de cheville définitive 1.

Selon l'invention, l'assemblage de la prothèse de cheville définitive 1 peut donc avantageusement être réalisé en dehors du champ opératoire, sur un poste de travail annexe, dans des conditions particulièrement aisées et pratiques, notamment en termes d'accessibilité, d'éclairage, de propreté, etc.

Par ailleurs, selon une variante de réalisation du kit 30 conforme à l'invention, l'implant talien définitif 2 servira également d'implant d'essai 102, sa mise en place directe limitant avantageusement le traumatisme subi par le talus en épargnant à ce dernier le remplacement d'un implant par l'autre. Par souci de clarté, on fera néanmoins référence dans la présente description à un « *implant talien d'essai 102* » pour désigner l'implant talien définitif 2 lorsque ce dernier est utilisé en tant qu'élément de la prothèse de cheville d'essai 101 pour évaluer la configuration selon laquelle la prothèse de cheville définitive 1 doit être implantée.

Il est également remarquable qu'en dotant l'implant tibial d'essai 104 de structures de centrage médio-latéral et antéro-postérieur homologues à celles de l'implant tibial définitif 4, à savoir respectivement un bossage (non représenté) et un bouclier tibial 123, il est possible de positionner immédiatement l'implant tibial définitif 4 par rapport au tibia 5 dans une position identique à celle précédemment occupée par l'implant tibial d'essai 104.

Bien entendu, le kit chirurgical conforme à l'invention n'est pas limité à l'implantation d'une prothèse de cheville et peut être décliné sous d'autres formes dans le cadre de la mise en oeuvre d'autres prothèses, notamment articulaires. L'homme du métier sera naturellement à même d'apprécier le dimensionnement et la géométrie des éléments constitutifs de la prothèse définitive et de la prothèse d'essai résultant d'une telle adaptation.

On peut prévoir également une méthode chirurgicale destinée à la mise en place chez un patient d'une prothèse de cheville 1 dite « *prothèse de cheville définitive* », ladite prothèse de cheville définitive 1 comprenant un implant talien 2 destiné à être implanté dans ou sur le talus 3, un implant tibial 4 destiné à être implanté dans ou sur le tibia 5 ainsi qu'un implant intermédiaire 6 destiné à être interposé entre ledit implant tibial et ledit implant talien, ledit implant intermédiaire 6 étant en outre conçu pour être monté mobile relativement audit implant talien 2 au niveau d'une interface de contact 7 afin d'autoriser une mobilité de la cheville.

De façon particulièrement préférentielle, ladite méthode pourra être mise en oeuvre à l'aide d'un kit chirurgical 30 tel que décrit précédemment auquel il sera fait référence à titre d'exemple particulier.

Ladite méthode chirurgicale comporte une étape (a) d'implantation d'une prothèse de cheville d'essai au cours de laquelle on implante au patient, en lieu et place de la prothèse de cheville définitive, une prothèse de cheville d'essai 101 comprenant un implant talien d'essai 102, un implant tibial d'essai 104, un implant intermédiaire d'essai 106.

Plus précisément, après avoir préparé les surfaces osseuses concernées, notamment par résection, le praticien rapporte et fixe provisoirement l'implant talien d'essai 102 dans ou sur le talus 3 et l'implant tibial d'essai 104 dans ou sur le tibia 5. Il positionne ensuite grossièrement le patin 106, 110 au sein de l'articulation, en intercalant ce dernier entre l'implant tibial d'essai 104 et l'implant talien d'essai 102, de telle sorte que d'une part sa surface 106A vienne au contact de la surface 102A de l'implant talien d'essai de manière à pouvoir y glisser avec frottement et, d'autre part, que sa surface opposée vienne en appui-plan libre contre l'implant tibial d'essai 104 au niveau de la zone de contact 108.

La méthode chirurgicale comporte également une étape (b) de détermination au cours de laquelle on détermine *in vivo* une configuration spécifique de ladite prothèse de cheville d'essai parmi une pluralité de configurations possibles.

De préférence, au cours de l'étape (b) de détermination, on identifie empiriquement, en tant que configuration spécifique de la prothèse de cheville d'essai 101, une configuration dite « *configuration neutre* » dans laquelle les contraintes exercées sur la prothèse de cheville d'essai 101 lors des mouvements naturels de l'articulation de la cheville sont globalement minimisées.

De façon particulièrement préférentielle, l'étape (b) de détermination de la configuration spécifique comprend une sous-étape (b1) de manipulation du patient au cours de laquelle on place ledit patient dans une posture prédéterminée de manière à pouvoir observer le comportement de la prothèse de cheville d'essai 101 sous l'effet de ladite manipulation, ainsi qu'une sous-étape (b2) de réglage au cours de laquelle on modifie la configuration de la prothèse de cheville d'essai 101 en fonction du comportement observé lors de la sous-étape (b1).

Plus particulièrement, le praticien peut forcer le mouvement du pied par rapport à la jambe, notamment en provoquant une flexion plantaire suivie d'une flexion dorsale, afin d'observer la réponse du patin 106, 110 à ces sollicitations.

De préférence, au cours de l'étape (b) de détermination, on réalise successivement plusieurs sous-étapes de manipulation (b1) suivies chacune d'une sous-étape de réglage (b2) de manière à régler itérativement la configuration de la prothèse de cheville d'essai 101. Le praticien peut notamment faire passer le patient cycliquement par plusieurs postures distinctes selon une séquence déterminée, et par exemple alterner de façon répétitive flexions dorsales et flexions plantaires.

Avantageusement, sous l'effet des manipulations de l'articulation, le patin 106, 110 tend spontanément à se centrer sensiblement sur la position P₀ dans laquelle les contraintes qu'il subit lors des déplacements de l'articulation sont sensiblement minimales.

En d'autres termes, les sous-étapes de manipulation (b1) et de réglage (b2) sont de préférence confondues, la prothèse de cheville d'essai 101 étant conçue pour s'auto-configurer en réponse à une ou plusieurs manipulations du patient.

Ainsi, le chirurgien peut, en faisant migrer itérativement le patin 106, 110 sous la contrainte par des manipulations successives du pied par rapport à la jambe, faire converger progressivement la prothèse de cheville d'essai 101, et plus particulièrement ledit patin 106, 110, vers sa configuration neutre.

La méthode chirurgicale comporte en outre une étape (c) de reproduction au cours de laquelle on reproduit sur la prothèse de cheville définitive 1 la configuration spécifique choisie lors de l'étape (b) de détermination en agençant l'implant intermédiaire 6 par rapport à l'implant tibial 4 à l'aide d'un moyen d'accouplement configurable 10.

De préférence, l'ensemble de la méthode étant appliqué au cours d'une unique intervention chirurgicale, celle-ci comporte une étape (d) de remplacement au cours de laquelle on extrait la prothèse de cheville d'essai 101 et l'on implante à sa place la prothèse de cheville définitive 1 placée dans la configuration spécifique choisie.

A ce titre, il est remarquable que ladite étape (d) de remplacement peut concerner tout ou partie de la prothèse de cheville d'essai, l'implant talien d'essai 102 pouvant par exemple, le cas échéant, être conservé et employé comme implant talien définitif 2.

De préférence, la méthode chirurgicale comporte une étape (e) de préparation de la prothèse de cheville d'essai 101 au cours de laquelle on associe à la prothèse de cheville d'essai un moyen de mesure 32 conçu pour permettre au praticien de relever la configuration de la prothèse de cheville d'essai 101 alors que cette dernière se trouve implantée au sein du patient.

L'étape (e) de préparation peut avantageusement être réalisée antérieurement à l'étape (a) d'implantation de la prothèse de cheville d'essai 101, de manière à pouvoir suivre en continu les évolutions de la configuration de ladite prothèse de cheville d'essai et recueillir à tout moment les données caractéristiques du positionnement de l'implant intermédiaire d'essai 106 par rapport à l'implant tibial d'essai 104.

De préférence, le moyen de mesure 32 étant pourvu d'un ou plusieurs organes tactiles 33, 34 conçus pour venir palper la prothèse de cheville d'essai 101, l'étape (c) de reproduction comprend une sous-étape (c1) de verrouillage du moyen de mesure 32 au cours de laquelle on fige la position desdits organes tactiles 33, 34 à l'aide d'un moyen de blocage réversible.

Plus précisément, le moyen de mesure 32 comportant un premier organe tactile 33 présentant une première surface de palpation 33A et un second organe tactile 34 présentant une seconde surface de palpation 34A destinées à venir respectivement au contact de l'implant tibial d'essai 104 et de l'implant intermédiaire d'essai 106, la sous-étape (c1) de verrouillage est effectuée en fixant le premier et le second organe tactile l'un à l'autre.

De façon particulièrement préférentielle, le praticien immobilise les rallonges parallélépipédiques formant lesdits organes tactiles 33, 34 en les comprimant l'une contre l'autre à l'aide d'une pince-étau lorsqu'il estime que la prothèse de cheville d'essai 101 a atteint la configuration neutre sous l'effet des manipulations effectuées lors de l'étape de détermination (b). II. peut ainsi effectuer un relevé de configuration et conserver durablement les informations recueillies.

Avantageusement, la mise en oeuvre du moyen de mesure 32 n'interfère pas avec la prothèse de cheville d'essai 101, et notamment ne provoque pas de déplacement accidentel incontrôlé du patin 106, 110 une fois la configuration neutre atteinte.

L'étape (c) de reproduction comporte de préférence également une sous-étape (c2) d'ajustement au cours de laquelle on configure la prothèse de cheville définitive 1 à l'aide d'un gabarit d'assemblage établi sur la base des données recueillies par le moyen de mesure 32.

Plus précisément, la sous-étape (c2) d'ajustement peut comprendre une première phase (c'2), postérieure à l'étape (c1) de verrouillage, au cours de laquelle on sépare l'ensemble formé par les organes tactiles 33, 34, toujours fixés l'un à l'autre, de la prothèse de cheville d'essai 101, ainsi qu'une deuxième phase (c"2) au cours de laquelle on rapporte respectivement l'implant tibial 4 définitif en appui contre la première surface de palpation 33A ainsi que le moyen d'accouplement 10, et plus particulièrement l'embase 12 de ce dernier, contre la seconde surface de palpation 34A, les organes tactiles formant ainsi le gabarit d'assemblage de la prothèse de cheville définitive 1, tel que cela est illustré sur la figure 5.

Avantageusement, la configuration de la prothèse de cheville définitive 1 peut donc être réalisée avant son implantation, sur un poste de travail dégagé.

De préférence, lors de la deuxième phase (c"2), le praticien accole l'embase 12 contre l'implant tibial 4 au niveau de la zone de contact 8 et engage les vis 16 sans les serrer, puis applique leur surface de repérage respective 4A, 12A contre les organes tactiles 33, 34 formant gabarit d'assemblage afin d'obtenir leur mise en place spontanée. Une fois l'embase 12 positionnée, le praticien serre les vis 16 puis insère l'implant intermédiaire 6 dans l'embase 12 en l'enfilant en butée dans la queue d'aronde.

Ainsi, le praticien peut transférer aisément à la prothèse de cheville définitive 1 une configuration spécifique propre au patient, déterminée empiriquement sur la prothèse de cheville d'essai 101 pour être le plus en harmonie possible avec la conformation articulaire dudit patient.

Bien entendu, la présente méthode chirurgicale ne se limite nullement à l'implantation d'une prothèse de cheville.

En particulier, elle peut constituer une méthode chirurgicale d'implantation chez un patient d'une prothèse dite « *prothèse définitive* », ladite méthode comportant une étape (K) d'implantation d'une prothèse d'essai au cours de laquelle on implante au patient, en lieu et place de la prothèse définitive, une prothèse d'essai qui reproduit sensiblement la géométrie de la prothèse définitive, une étape (L) de détermination au cours de laquelle on détermine *in vivo* une configuration fonctionnelle particulière de ladite prothèse d'essai parmi une pluralité de configurations possibles, ainsi qu'une étape (M) de reproduction au cours de laquelle on reproduit sur la prothèse définitive la configuration fonctionnelle particulière choisie lors de l'étape (L) de détermination à l'aide de moyens d'ajustement dont est pourvue ladite prothèse définitive.

Par « *configuration fonctionnelle* », on entend à la fois la disposition spatiale statique (forme, volume, position et orientation des différents éléments constitutifs de la prothèse) et l'agencement cinématique (degrés de liberté) qui seront mis en oeuvre dans le cadre du fonctionnement normal de la prothèse définitive.

Au sens de l'invention, la prothèse définitive et la prothèse d'essai peuvent adopter chacune intrinsèquement plusieurs configurations fonctionnelles, chaque configuration fonctionnelle de la prothèse définitive possédant son homologue (c'est-à-dire, selon la notion d'ensembles mathématiques, « *un antécédent* ») sur la prothèse d'essai. La méthode vise donc à « *régler* » la prothèse définitive en réalisant au préalable une mise en situation de la prothèse d'essai afin de rechercher la configuration fonctionnelle la mieux appropriée.

A cet effet, la prothèse d'essai conforme à l'invention reproduit pour l'essentiel la géométrie et/ou les liaisons cinématiques de la prothèse définitive. Elle est de plus destinée à être implantée puis remplacée par la prothèse définitive au cours d'une seule et même intervention.

Ladite méthode chirurgicale est tout particulièrement applicable à la mise en place au sein d'une articulation d'une prothèse articulaire visant à assurer une certaine mobilité de ladite articulation.

De façon particulièrement préférentielle, au cours de l'étape (L) de détermination, on identifie alors empiriquement, en tant que configuration fonctionnelle particulière de la prothèse d'essai, une configuration dite « *configuration neutre* » dans laquelle les contraintes exercées sur la prothèse d'essai lors des mouvements naturels de l'articulation sont globalement minimisées.

De préférence, l'étape (L) de détermination comprend à cet effet une sous-étape (L1) de manipulation du patient au cours de laquelle on place ledit patient dans une posture prédéterminée de manière à pouvoir observer le comportement de la prothèse d'essai sous l'effet de ladite manipulation, ainsi qu'une sous-étape (L2) de réglage au cours de laquelle on modifie la configuration de la prothèse d'essai en fonction du comportement observé lors de la sous-étape (L1) de manipulation.

De préférence, ladite méthode comporte en outre une étape (O) de préparation de la prothèse d'essai au cours de laquelle on associe à la prothèse d'essai un moyen de mesure conçu pour permettre au praticien de relever la configuration de la prothèse d'essai alors que cette dernière se trouve implantée au sein du patient.

Ainsi, de façon particulièrement avantageuse, la prothèse 1 conforme à l'invention présente un excellent compromis entre la stabilité de l'articulation qui facilite la rééducation du patient, notamment en cas de laxité des tendons ou d'atrophie des muscles, le confort d'utilisation lié notamment à la moindre sensation d'entrave, et la longévité du fait que ladite prothèse est faiblement sujette à l'usure.

En outre, il est possible de régler la prothèse conforme à l'invention grâce à un unique moyen d'accouplement, sans qu'il soit nécessaire par exemple de recourir à un jeu d'implants interchangeables de tailles et de formes différentes que le chirurgien teste itérativement en les implantant les uns après les autres jusqu'à trouver une combinaison satisfaisante. La mise en oeuvre d'une prothèse, et plus globalement d'un kit chirurgical conformes à l'invention permet donc de réaliser de substantielles économies de matériau et de temps d'intervention, tout en limitant les risques opératoires liés à la multiplication des gestes liés aux essais *in vivo.*

Ainsi, la prothèse de cheville conforme à l'invention permet avantageusement une personnalisation du traitement orthopédique vis-à-vis de chaque patient, tout en conservant une structure simple et une mise en oeuvre particulièrement facile et fiable.

## Revendications

1. Prothèse de cheville (1) comprenant un implant talien (2) destiné à être implanté dans ou sur le talus (3), un implant tibial (4) destiné à être implanté dans ou sur le tibia (5) ainsi qu'un implant intermédiaire (6) destiné à être interposé entre ledit implant tibial et ledit implant talien, ledit implant intermédiaire (6) étant en outre conçu pour être monté mobile relativement audit implant talien (2) au niveau d'une interface de contact (7) afin d'autoriser une mobilité de la cheville, ladite prothèse (1) étant pourvue d'un moyen d'accouplement configurable (10) embarqué conçu pour autoriser le réglage de la position dudit implant intermédiaire (6) par rapport audit implant tibial (4), ladite prothèse de cheville (1) étant **caractérisée en ce que** ledit moyen d'accouplement configurable (10) permet d'une part d'ajuster la position de l'implant intermédiaire (6) par rapport à l'implant tibial (4) angulairement et linéairement dans un plan sensiblement normal à l'axe médullaire (ZZ') du tibia afin de permettre à la prothèse (1) d'adopter intrinsèquement plusieurs configurations fonctionnelles possibles, et d'autre part de sélectionner l'agencement de l'implant intermédiaire (6) par rapport à l'implant tibial (4) selon une configuration spécifique choisie parmi cette pluralité de configurations fonctionnelles possibles.

2. Prothèse de cheville selon la revendication 1 **caractérisée en ce que** la configuration spécifique est dite « *configuration neutre* » et correspond à une position de l'implant intermédiaire (6) relativement à l'implant tibial (4) dans laquelle ledit implant intermédiaire (6) est sensiblement centré par rapport au point d'origine (P₀) autour duquel s'effectuent les débattements du tibia (5) par rapport au talus (3) en translation antéro-postérieure, en translation médio-latérale et en rotation autour de l'axe médullaire (ZZ') du tibia (5), et dans laquelle l'implant tibial (4) et/ou l'implant intermédiaire (6) sont orientés de telle sorte que les axes cinématiques de la prothèse (1) coïncident sensiblement avec les axes anatomiques naturels de la cheville du patient.

3. Prothèse de cheville selon l'une des revendications 1 ou 2 **caractérisée en ce que** le moyen d'accouplement configurable (10) comporte des moyens d'encastrement (11) de l'implant intermédiaire (6) sur l'implant tibial (4).

4. Prothèse de cheville selon l'une des revendications précédentes **caractérisée en ce que** le moyen d'accouplement configurable (10) comporte une embase (12) conçue pour être fixée sur l'implant tibial (4) et former une interface mécanique entre ledit implant tibial (4) et l'implant intermédiaire (6).

5. Prothèse de cheville selon la revendication 4 **caractérisée en ce que** l'embase (12) dispose de moyens de fixation réglables (14) qui permettent de choisir, parmi une pluralité de positions possibles, la position dans laquelle ladite embase (12) est effectivement fixée par rapport à l'implant tibial (4).

6. Prothèse de cheville selon la revendication 5 **caractérisée en ce que** les moyens de fixation réglables (14) forment un moyen de réglage continu de la position de fixation de l'embase (12).

7. Prothèse de cheville selon l'une des revendications 4 à 6 **caractérisée en ce que** l'embase (12) est pourvue de moyens d'accueil (20) permettant le raccordement de l'implant intermédiaire (6) à ladite embase (12), lesdits moyens d'accueil (20) étant agencés de telle sorte que l'implant intermédiaire (6) ne puisse occuper qu'une position unique et prédéterminée par rapport à ladite embase (12).

8. Prothèse de cheville selon l'une des revendications 4 à 7 **caractérisée en ce que** l'embase (12) se trouve en appui-plan contre l'implant tibial (4).

9. Prothèse de cheville selon l'une des revendications précédentes **caractérisée en ce qu'**elle comprend un moyen de repérage (22) conçu pour indiquer l'agencement de l'implant intermédiaire (6) par rapport à l'implant tibial (4) afin de permettre au praticien de configurer précisément le moyen d'accouplement configurable (10).

10. Prothèse de cheville selon l'une des revendications 4 à 8 et la revendication 9, **caractérisée en ce que** le moyen de repérage (22) comprend une première surface de repérage (4A) associée à l'implant tibial (4) et une seconde surface de repérage (12A) associée à l'embase (12), la position relative desdites première et seconde surfaces de repérage permettant ainsi de déterminer la position de ladite embase (12) dans le repère que forme l'implant tibial (4).

11. Procédé de fabrication en usine d'une prothèse de cheville comprenant un implant talien (2) destiné à être implanté dans ou sur le talus (3), un implant tibial (4) destiné à être implanté dans ou sur le tibia (5) ainsi qu'un implant intermédiaire (6) destiné à être interposé entre ledit implant tibial et ledit implant talien, ledit implant intermédiaire (6) étant conçu pour être monté mobile relativement audit implant talien (2) au niveau d'une interface de contact (7) afin d'autoriser une mobilité de la cheville, **caractérisé en ce qu'**il comporte une étape de réalisation d'un moyen d'accouplement configurable (10) au cours de laquelle on réalise un moyen d'accouplement configurable (10) embarqué conçu d'une part pour autoriser le réglage de la position dudit implant intermédiaire (6) par rapport audit implant tibial (4) angulairement et linéairement dans un plan sensiblement normal à l'axe médullaire (ZZ') du tibia afin de permettre à la prothèse (1) d'adopter intrinséquement plusieurs configurations fonctionnelles possibles, et d'autre part pour permettre la sélection de l'agencement de l'implant intermédiaire (6) par rapport à l'implant tibial (4) selon une configuration spécifique choisie parmi cette pluralité de configurations fonctionnelles possibles.

12. Procédé de fabrication selon la revendication 11 **caractérisé en ce qu'**il comporte une étape d'assemblage au cours de laquelle on assemble l'implant intermédiaire (6) et l'implant tibial (4) en agençant en usine ledit implant intermédiaire (6) par rapport audit implant tibial (4) à l'aide du moyen d'accouplement configurable (10).

13. Prothèse de cheville d'essai (101) **caractérisée en ce qu'**elle est conçue pour être implantée provisoirement en lieu et place d'une prothèse de cheville définitive conforme à l'une des revendications 1 à 10, ladite prothèse définitive (1) pouvant être configurée selon une configuration spécifique choisie parmi une pluralité de configurations possibles par un moyen d'accouplement configurable (10) permettant un ajustement angulaire et linéaire dans un plan sensiblement normal à l'axe médullaire (ZZ') du tibia, et **en ce que** ladite prothèse de cheville d'essai (101) comprend un implant talien d'essai (102), un implant tibial d'essai (104), un implant intermédiaire d'essai (106) et un moyen d'accouplement configurable d'essai (110) dont les formes et les dimensions correspondent sensiblement à celles de l'implant talien (2), de l'implant implant tibial (4), de l'implant intermédiaire (6) et du moyen d'accouplement configurable (10) de la prothèse définitive (1) respectivement, de manière à ce que ladite prothèse d'essai (101) puisse adopter *in vivo* des configurations fonctionnelles homologues aux configurations fonctionnelles possibles de la prothèse définitive.

14. Prothèse de cheville d'essai (101) selon la revendication 13 **caractérisé en ce qu'**elle est pourvue de moyens de repérage (122) agencés pour permettre au praticien de relever la configuration dans laquelle elle se trouve de manière à pouvoir reproduire celle-ci sur la prothèse de cheville définitive.

15. Prothèse de cheville d'essai selon la revendication 14 **caractérisée en ce que** les moyens de repérage (122) sont agencés pour coopérer avec les organes tactiles (33, 34) d'un moyen de mesure (32) conçu pour permettre au praticien de relever la configuration de la prothèse de cheville d'essai (101) alors que cette dernière se trouve implantée au sein du patient.

16. Prothèse de cheville d'essai selon la revendication 14 ou 15 **caractérisée en ce que** les moyens de repérage (122) sont agencés pour être solidarisés avec lesdits organes tactiles (33, 34) avant implantation de ladite prothèse de cheville d'essai de manière à pouvoir coopérer avec ces derniers pendant que la prothèse de cheville d'essai est implantée.

17. Prothèse de cheville d'essai selon l'une des revendications 13 à 16 **caractérisée en ce que** l'implant intermédiaire d'essai (106) et le moyen d'accouplement configurable d'essai (110) forment un ensemble monobloc de type patin.

18. Prothèse de cheville d'essai selon l'une des revendications 13 à 17 **caractérisée en ce qu'**elle est pourvue de moyens d'auto-centrage agencés pour amener ladite prothèse de cheville d'essai dans une configuration neutre, dans laquelle les contraintes exercées sur l'implant intermédiaire d'essai (106) et l'implant tibial d'essai (104) lors des mouvements naturels de la cheville soient globalement minimisées.

19. Prothèse de cheville d'essai selon les revendications 17 et 18 **caractérisée en ce que** les moyens d'auto-centrage sont formés par le patin (106, 110) qui est agencé pour être interposé librement entre l'implant talien d'essai (102) et l'implant tibial d'essai (104) de manière à se trouver d'une part en liaison appui-plan avec ce dernier, et d'autre part en liaison pivot glissant avec l'implant talien d'essai (102).

20. Kit chirurgical (30) destiné à la mise en place d'une prothèse de cheville, **caractérisé en ce qu'**il comporte une prothèse de cheville (1) selon l'une des revendications 1 à 10, dite **«** *prothèse de cheville définitive* », ainsi qu'une prothèse de cheville d'essai (101) selon l'une des revendications 13 à 19.

21. Kit chirurgical selon la revendication 20 **caractérisé en ce qu'**il comprend un moyen de mesure (32) conçu pour permettre au praticien de relever la configuration de la prothèse de cheville d'essai (101) alors que cette dernière se trouve implantée au sein du patient.

22. Kit chirurgical selon la revendication 21 **caractérisé en, ce que** le moyen de mesure (32) est conçu pour relever la position de l'implant intermédiaire d'essai (106) par rapport à l'implant tibial d'essai (104).

23. Kit chirurgical selon la revendication 22 **caractérisé en ce que** le moyen de mesure (32) comporte un premier organe tactile (33) présentant une première surface de palpation (33A) et un second organe tactile (34) présentant une seconde surface de palpation (34A) destinées à venir respectivement au contact de l'implant tibial d'essai (104) et de l'implant intermédiaire d'essai (106).

24. Kit chirurgical selon la revendication 23 **caractérisé en ce que** le premier et le second organe tactile (33, 34) sont pourvus de moyens d'association respectivement à l'implant tibial d'essai (104) et à l'implant intermédiaire d'essai (106) de manière à pouvoir d'une part être solidarisés à ces derniers avant l'implantation de la prothèse de cheville d'essai (101) et d'autre part rester solidaires de ces derniers pendant que ladite prothèse de cheville d'essai (101) se trouve dans le patient.

25. Kit chirurgical selon la revendication 23 ou 24 **caractérisé en ce que** le premier et le second organe tactile sont formés respectivement par une première et une seconde rallonge conçues pour présenter chacune, lorsque la prothèse de cheville d'essai (101) est implantée, une extrémité qui dépasse hors de l'incision pratiquée pour accéder à la cheville du patient.

26. Kit chirurgical selon l'une des revendications 23 à 25 **caractérisé en ce que** le premier et le second organe tactile (33, 34) sont liés par un moyen de blocage réversible ou amovible de manière à ce qu'ils puissent être laissés mobiles l'un par rapport à l'autre ou au contraire solidarisés l'un à l'autre.

27. Kit chirurgical selon l'une des revendications 21 à 26 **caractérisé en ce que** le moyen de mesure (32) est également conçu pour permettre le report de la configuration de la prothèse de cheville d'essai (101) sur la prothèse de cheville définitive (1), ledit moyen de mesure (32) formant ainsi un gabarit d'assemblage de ladite prothèse de cheville définitive (1).

## Claims

1. An ankle prosthesis (1) comprising a talar implant (2) designed to be implanted in or on the talus (3), a tibial implant (4) designed to be implanted in or on the tibia (5), and an intermediate implant (6) designed to be interposed between the tibial implant (4) and the talar implant (2), said intermediate implant (6) being further designed to be mounted to move relative to said talar implant (2) at a contact interface (7) in order to allow the ankle to move, said ankle prosthesis (1) being provided with on-board configurable coupling means (10) designed to enable the position of said intermediate implant (6) to be adjusted relative to the tibial implant (4), said ankle prosthesis being **characterized in that** said configurable coupling means (10) enable firstly the position of the intermediate implant (6) relative to the tibial implant (4) to be adjusted angularly and linearly in a plane that is substantially perpendicular to the medullary axis (ZZ') of the tibia so as to allow the prosthesis (1) intrinsically to adopt a plurality of possible functional configurations, and secondly the arrangement of the intermediate implant (6) relative to the tibial implant (4) to be selected to be a specific configuration chosen from said plurality of possible functional configurations.

2. An ankle prosthesis according to claim 1,
**characterized in that** the specific configuration is referred to as the "neutral configuration" and corresponds to the intermediate implant (6) being in a position relative to the tibia implant (4) firstly in which said intermediate implant (6) is substantially centered relative to the point of origin (Pₒ) about which the movements of the tibia (5) relative to the talus (2) in anteroposterior translation, in mediolateral translation, and in rotation about the medullary axis (ZZ') of the tibia (5) take place, and secondly in which the tibial implant (4) and/or the intermediate implant (6) is/are oriented such that the movement axes of the prosthesis (1) substantially coincide with the natural anatomical axes of the ankle of the patient.

3. An ankle prosthesis according to claim 1 or claim 2, **characterized in that** the configurable coupling means (10) preferably include engagement means (11) for engaging the intermediate implant (6) into the tibial implant (4).

4. An ankle prosthesis according to any preceding claim, **characterized in that** the configurable coupling means (10) include a base (12) designed to be fastened to the tibial implant (4) and to form a mechanical interface between said tibial implant (4) and the intermediate implant (6).

5. An ankle prosthesis according to claim 4, **characterized in that** the base (12) is provided with adjustable fastening means (14) which make it possible to choose, from among a plurality of possible positions, that position in which said base (12) is actually fastened relative to the tibial implant (4).

6. An ankle prosthesis according to claim 5, **characterized in that** the adjustable fastening means (14) form means for continuously adjusting the fastening position of the base (12).

7. An ankle prosthesis according to any one of claims 4 to 6, **characterized in that** the base (12) is provided with reception means (20) making it possible to connect the intermediate implant (6) to said base (12), said reception means (20) being arranged such that the intermediate implant (6) can occupy a single and predetermined position only relative to the base (12).

8. An ankle prosthesis according to any one of claims 4 to 7, **characterized in that** the base (12) finds itself in plane abutment against the tibial implant (4).

9. An ankle prosthesis according to any preceding claim, **characterized in that** it further comprises reference means (22) designed to indicate how the intermediate implant (6) is arranged relative to the tibial implant (4) in order to enable the practitioner to configure the configurable coupling means (10) accurately.

10. An ankle prosthesis according to any one of claims 4 to 8, and claim 9, **characterized in that** the reference means (22) comprise a first reference surface (4A) associated with the tibial implant (4) and a second reference surface (12A) associated with the base (12), the relative position of said first and second reference surfaces thus making it possible to determine the position of said base (12) in the frame of reference formed by the tibial implant (4).

11. A method of fabricating an ankle prosthesis in a factory, the prosthesis comprising a talar implant (2) designed to be implanted in or on the talus (3), a tibial implant (4) designed to be implanted in or on the tibia (5), and an intermediate implant (6) designed to be interposed between said tibial implant and said talar implant, said intermediate implant (6) being designed to be mounted to move relative to said talar implant (2) at a contact interface (7) in order to allow the ankle to move, the method being **characterized in that** it includes a step of making a configurable coupling means (10) during which an on-board configurable coupling means (10) is made that is designed firstly to enable the position of said intermediate implant (6) relative to the tibial implant (4) to be adjusted angularly and linearly in a plane that is substantially perpendicular to the medullary axis (ZZ') of the tibia so as to allow the prosthesis (1) intrinsically to adopt a plurality of possible functional configurations, and secondly to enable the arrangement of the intermediate implant (6) relative to the tibial implant (4) to be selected to be a specific configuration chosen from said plurality of possible functional configurations.

12. A fabrication method according to claim 11, **characterized in that** it includes an assembly step during which the intermediate implant (6) and the tibial implant (4) are assembled together by arranging in the factory said intermediate implant (6) relative to said tibial implant (4) using the configurable coupling means (10).

13. A test ankle prosthesis (101), **characterized in that** it is designed to be implanted temporarily in place of a final ankle prosthesis according to any one of claims 1 to 10, said final prosthesis (1) being capable of being configured in a specific configuration chosen from among a plurality of possible configurations by a configurable coupling means (10) enabling an angular and linear adjustment in a plane that is substantially perpendicular to the medullary axis (ZZ') of the tibia, and **in that** said test ankle prosthesis (101) comprises a test talar implant (102), a test tibial implant (104), a test intermediate implant (106) and test configurable coupling means (110) of shapes and dimensions that correspond substantially to the shapes and dimensions respectively of the talar implant (2), of the tibial implant (4), of the intermediate implant (6), and of the configurable coupling means (10) of the final prosthesis (1), so that said test prosthesis can, in vivo, take up said functional configurations corresponding to possible functional configurations of the final prosthesis.

14. A test ankle prosthesis (101) according to claim 13, **characterized in that** it is provided with reference means (122) arranged to enable the practitioner to take the measurements of the configuration in which it finds itself so as to reproduce said configuration in the final ankle prosthesis.

15. A test ankle prosthesis according to claim 14, **characterized in that** the reference means (122) are arranged so as to co-operate with the touch-sensitive members (33, 34) of measurement means (32) designed to enable the practitioner to take the measurements of the configuration of the test ankle prosthesis (101) when said ankle prosthesis finds itself implanted in the patient.

16. A test ankle prosthesis according to claim 14 or claim 15, **characterized in that** reference means (122) are arranged so as to be secured to said touch-sensitive members (33, 34) prior to implantation of said test ankle prosthesis, so as to be capable of co-operating therewith while the test ankle prosthesis is implanted.

17. A test ankle prosthesis according to any one of claims 13 to 16, **characterized in that** the test intermediate implant (106) and the test configurable coupling means (110) form a one-piece unit of the block type.

18. A test ankle prosthesis according to any one of claims 13 to 17, **characterized in that** it is provided with self-centering means arranged so as to bring said test ankle prosthesis into a neutral configuration, in which the stresses exerted on the test intermediate implant (106) and on the test tibial implant (104) during natural movements of the ankle are minimized overall.

19. A test ankle prosthesis according to claims 17 and 18, **characterized in that** the self-centering means are formed by the block (106, 110) which is arranged so as to be interposed freely between the test talar implant (102) and the test tibial implant (104) in a manner such as to find itself firstly in plane abutment connection therewith, and secondly in sliding pivotal connection with the test talar implant (102).

20. A surgical kit (30) designed for putting an ankle prosthesis into place, said surgical kit being **characterized in that** it comprises an ankle prosthesis (1) according to any one of claims 1 to 10, referred to as a "final ankle prosthesis", and a test ankle prosthesis (101) according to any one of claims 13 to 19.

21. A surgical kit according to claim 20, **characterized in that** it further comprises measurement means (32) designed to enable the practitioner to take the measurements of the configuration of the test ankle prosthesis (101) while said test ankle prosthesis (101) is implanted in the patient.

22. A surgical kit according to claim 21, **characterized in that** the measurement means (32) are designed to take the measurements of the position of the test intermediate implant (106) relative to the test tibial implant (104).

23. A surgical kit according to claim 22, **characterized in that** the measurement means (32) comprise a first touch-sensitive member (33) presenting a first feeler surface (33A) and a second touch-sensitive member (34) presenting a second feeler surface (34A), the feeler surfaces being designed to come into contact respectively with the test tibial implant (104) and with the test intermediate implant (106).

24. A surgical kit according to claim 23, **characterized in that** the first and second touch-sensitive members (33, 34) are provided with association means for associating them respectively with the test tibial implant (104) and with the test intermediate implant (106) so that it is possible firstly for them to be secured to the respective ones of these implants prior to implantation of the test ankle prosthesis (101) and secondly for them to remain secured to said implants while said test ankle prosthesis (101) is inside the patient.

25. A surgical kit according to claim 23 or claim 24, **characterized in that** the first and second touch-sensitive members are formed respectively by first and second extension leaves, each of which is designed to have one end that, when the test ankle prosthesis (101) is implanted, projects from the incision made to access the ankle of the patient.

26. A surgical kit according to any one of claims 23 to 25, **characterized in that** the first and second touch-sensitive members (33, 34) are coupled together by reversible or releasable locking means so that they can be allowed to move relative to each other or else be constrained to move with each other.

27. A surgical kit according to any one of claims 21 to 26, **characterized in that** the measurement means (32) are also designed to enable the configuration of the test ankle prosthesis (101) to be transposed to the final ankle prosthesis (1), said measurement means (32) thus forming an assembly template for said final ankle prosthesis (1).

## Patentansprüche

1. Knöchelprothese (1), umfassend ein Fersenimplantat (2), das dazu bestimmt ist, in oder auf die Ferse (3) implantiert zu werden, ein Schienbeinimplantat (4), das dazu bestimmt ist, in oder auf das Schienbein (5) implantiert zu werden, sowie ein Zwischenimplantat (6), das dazu bestimmt ist, zwischen dem Schienbeinimplantat und dem Fersenimplantat angeordnet zu werden, wobei das Zwischenimplantat (6) ferner dazu geeignet ist, in Bezug zum Fersenimplantat (2) im Bereich einer Kontaktschnittstelle (7) beweglich montiert zu werden, um eine Beweglichkeit des Knöchels zu gestatten, wobei die Prothese (1) mit einem konfigurierbaren eingebauten Kuppelmittel (10) versehen ist, das dazu geeignet ist, die Einstellung der Position des Zwischenimplantats (6) in Bezug zum Schienbeinimplantat (4) zu gestatten, wobei die Knöchelprothese (1) **dadurch gekennzeichnet ist, dass** es das konfigurierbare Kuppelmittel (10) ermöglicht, einerseits die Position des Zwischenimplantats (6) in Bezug zum Schienbeinimplantat (4) im Winkel und linear in einer Ebene im Wesentlichen normal auf die Markachse (ZZ') des Schienbeins einzustellen, um es der Prothese (1) zu ermöglichen im Inneren mehrere mögliche Funktionskonfigurationen anzunehmen, und andererseits die Anordnung des Zwischenimplantats (6) in Bezug zum Schienbeinimplantat (4) in einer spezifischen Konfiguration, die unter dieser Vielzahl von möglichen Funktionskonfigurationen ausgewählt wird, zu wählen.

2. Knöchelprothese nach Anspruch 1, **dadurch gekennzeichnet, dass** die spezifische Konfiguration *"neutrale* Konfiguration" genannt wird und einer Position des Zwischenimplantats (6) in Bezug zum Schienbeinimplantat (4) entspricht, in der das Zwischenimplantat (6) im Wesentlichen in Bezug zu dem Ausgangspunkt (P₀) zentriert ist, um den die Bewegungen des Schienbeins (5) in Bezug zur Ferse (3) in anteroposteriorer Translation, in mediolateraler Translation und in Rotation um die Markachse (ZZ') des Schienbeins (5) erfolgen, und in der das Schienbeinimplantat (4) und/oder das Zwischenimplantat (6) derart ausgerichtet sind, dass die kinematischen Achsen der Prothese (1) im Wesentlichen mit den natürlichen anatomischen Achsen des Knöchels des Patienten zusammenfallen.

3. Knöchelprothese nach einem der Ansprüche 1 oder 2, **dadurch gekennzeichnet, dass** das konfigurierbare Kuppelelement (10) Mittel (11) zum Einsetzen des Zwischenimplantats (6) auf das Schienbeinimplantat (4) umfasst.

4. Knöchelprothese nach einem der vorhergehenden Ansprüche, **dadurch gekennzeichnet, dass** das konfigurierbare Kuppelmittel (10) ein Basis (12) umfasst, die dazu geeignet ist, auf dem Schienbeinimplantat (4) befestigt zu werden und eine mechanische Schnittstelle zwischen dem Schienbeinimplantat (4) und dem Zwischenämplantat (6) zu bilden.

5. Knöchelprothese nach Anspruch 4, **dadurch gekennzeichnet, dass** die Basis (12) über einstellbare Befestigungsmittel (14) verfügt, die es ermöglichen, unter einer Vielzahl von möglichen Positionen die Position auszuwählen, in der die Basis (12) tatsächlich in Bezug zum Schienbeinimplantat (4) fixiert ist.

6. Knöchelprothese nach Anspruch 5, **dadurch gekennzeichnet, dass** die einstellbaren Befestigungsmittel (14) ein Mittel zur kontinuierlichen Einstellung der Befestigungsposition der Basis (12) bilden.

7. Knöchelprothese nach einem der Ansprüche 4 bis 6, **dadurch gekennzeichnet, dass** die Basis (12) mit Aufnahmemitteln (20) versehen ist, die den Anschluss des Zwischenimplantats (6) an die Basis (12) ermöglichen, wobei die Aufnahmemittel (20) derart vorgesehen sind, dass das Zwischenimplantat (6) nur eine einzige und vorbestimmte Position in Bezug zur Basis (12) einnehmen kann.

8. Knöchelprothese nach einem der Ansprüche 4 bis 7, **dadurch gekennzeichnet, dass** die Basis (12) flach am Schienbeinimplantat (4) anliegt.

9. Knöchelprothese nach einem der vorhergehenden Ansprüche, **dadurch gekennzeichnet, dass** sie ein Ortungsmittel (22) umfasst, das dazu geeignet ist, die Anordnung des Zwischenimplantats (6) in Bezug zum Schienbeinimplantat (4) anzuzeigen, um es dem Arzt zu ermöglichen, das konfigurierbare Kuppelmittel (10) genau zu konfigurieren.

10. Knöchelprothese nach einem der Ansprüche 4 bis 8 und Anspruch 9, **dadurch gekennzeichnet, dass** das Ortungsmittel (22) eine erste Ortungsfläche (4A), die dem Schienbeinimplantat (4) zugeordnet ist, und eine zweite Ortungsfläche (12A), die der Basis (12) zugeordnet ist, umfasst, wobei es die relative Position der ersten und zweiten Ortungsflächen somit ermöglicht, die Position der Basis (12) in dem vom Schienbeinimplantat (4) gebildeten Ortungsbereich zu bestimmen.

11. Verfahren zur fabrikmäßigen Herstellung einer Knochelprothese, umfassend ein Fersenimplantat (2), das dazu bestimmt ist, in oder auf die Ferse (3) implantiert zu werden, ein Schienbeinimplantat (4), das dazu bestimmt ist, in oder auf das Schienbein (5) implantiert zu werden, sowie ein Zwischenimplantat (6), das dazu bestimmt ist, zwischen dem Schienbeinimplantat und dem Fersenimplantat angeordnet zu werden, wobei das Zwischenimplantat (6) dazu geeignet ist, in Bezug zum Fersenimplantat (2) im Bereich einer Kontaktschnittstelle (7) beweglich montiert zu werden, um eine Beweglichkeit des Knöchels zu gestatten, **dadurch gekennzeichnet, dass** es einen Schritt der Herstellung eines konfigurierbaren Kuppelmittels (10) umfasst, während dessen ein eingebautes konfigurierbares Kuppelmittel (10) hergestellt wird, das dazu geeignet ist, einerseits die Einstellung der Position des Zwischenimplantats (6) in Bezug zum Schienbeinimplantat (4) im Winkel und linear in einer Ebene im Wesentlichen normal zur Markachse (ZZ') des Schienbeins zu gestatten, um es der Prothese (1) zu ermöglichen, im Inneren mehrere mögliche Funktionskonfigurationen abzunehmen, und andererseits die Auswahl der Anordnung des Zwischenimplantats (6) in Bezug zum Schienbeinimplantat (4) in einer spezifischen Konfiguration, die unter dieser Vielzahl von möglichen Funktionskonfigurationen gewählt wird, zu ermöglichen.

12. Herstellungsverfahren nach Anspruch 11, **dadurch gekennzeichnet, dass** es einen Schritt des Zusammenbaus umfasst, während dessen das Zwischenimplantat (6) und das Schienbeinimplantat (4) zusammengebaut werden, wobei im Werk das Zwischenimplantat (6) in Bezug zum Schienbeinimplantat (4) mit Hilfe des konfigurierbaren Kuppelelements (10) angeordnet wird.

13. Probeknöchelprothese (101), **dadurch gekennzeichnet, dass** sie dazu vorgesehen ist, provisorisch an Ort und Stelle einer endgültigen Knöchelprothese nach einem der Ansprüche 1 bis 10 implantiert zu werden, wobei die endgültige Prothese (1) nach einer spezifischen Konfiguration ausgeführt sein kann, die unter einer Vielzahl von möglichen Konfigurationen durch ein konfigurierbares Kuppelmittel (10) ausgewählt wird, das eine Einstellung im Winkel und eine lineare Einstellung in einer Ebene im Wesentlichen normal auf die Markachse (ZZ') des Schienbeins ermöglicht, und dass die Probeknöchelprothese (101) ein Probefersenimplantat (102), ein Probeschienbeinimplantat (104), ein Probezwischenimplantat (106) und ein konfigurierbares Probekuppelmittel (110) umfasst, deren Formen und Abmessungen im Wesentlichen jenen des Fersenimplantats (2), des Schienbeinimplantats (4), des Zwischenimplantats (6) bzw. des konfigurierbaren Kuppelmittels (10) der endgültigen Prothese (1) entsprechen, so dass die Probeprothese (101) *in vivo* Funktionskonfigurationen annehmen kann, die mit den möglichen Funktionskonfigurationen der endgültigen Prothese gleichartig sind.

14. Probeknöchelprothese (101) nach Anspruch 13, **dadurch gekennzeichnet, dass** sie mit Ortungsmitteln (122) versehen ist, die derart vorgesehen sind, dass sie es dem Arzt ermöglichen, die Konfiguration, in der sie sich befindet, zu bestimmen, um diese auf der endgültigen Knöchelprothese zu reproduzieren.

15. Probeknochelprothese nach Anspruch 14, **dadurch gekennzeichnet, dass** die Ortungsmittel (122) derart vorgesehen sind, dass sie mit den Tastelementen (33, 34) eines Messhilfsmittels (32) zusammenwirken, das derart ausgeführt ist, dass es dem Arzt möglich ist, die Konfiguration der Probeknöchelprothese (101) zu bestimmen, wenn diese letztgenannte im Körper des Patienten implantiert ist.

16. Probeknöchelprothese nach Anspruch 14 oder 15, **dadurch gekennzeichnet, dass** die Ortungsmittel (122) derart vorgesehen sind, dass sie mit den Tastelementen (33, 34) vor der Implantation der Probeknöchelprothese verbunden werden, um mit diesen letztgenannten zusammenzuwirken, während die Probeknöchelprothese implantiert wird.

17. Probenknöchelprothese nach einem der Ansprüche 13 bis 16, **dadurch gekennzeichnet, dass** das Probezwischenimplantat (106) und das konfigurierbare Probekuppelmittel (110) eine einstückige Einheit vom Typ Kufe bilden.

18. Probeknöchelprothese nach einem der Ansprüche 13 bis 17, **dadurch gekennzeichnet, dass** sie mit Selbstzentriermitteln versehen ist, die derart vorgesehen sind, dass sie die Probeknöchelprothese in eine neutrale Konfiguration bringen, in der die auf das Probezwischenimplantat (106) und das Probeschienbeinimplantat (104) bei den natürlichen Bewegungen des Knöchels ausgeübten Spannungen insgesamt minimiert sind.

19. Probeknöchelprothese nach den Ansprüchen 17 und 18, **dadurch gekennzeichnet, dass** die Selbztzentriermittel von der Kufe (106, 110) gebildet sind, die derart vorgesehen ist, dass sie frei zwischen dem Probefersenimplantat (102) und dem Probeschienbeinimplantat (104) angeordnet werden kann, um einerseits flach anliegend mit diesem letztgenannten verbunden zu sein und andererseits mit dem Probefersenimplantat (102) schwenkend gleitend verbunden zu sein.

20. Chirurgenset (30), das für die Anbringung einer Knöchelprothese bestimmt ist, **dadurch gekennzeichnet, dass** es eine Knöchelprothese (1) nach einem der Ansprüche 1 bis 10, *"endgültige Knöchelprothese"* genannt, sowie eine Probeknöchelprothese (101) nach einem der Ansprüche 13 bis 19 umfasst.

21. Chirurgenset nach Anspruch 20, **dadurch gekennzeichnet, dass** es ein Messhilfsmittel (32) umfasst, das dazu vorgesehen ist, es dem Arzt zu ermöglichen die Konfiguration der Probeknöchelprothese (101) zu bestimmen, wenn diese im Körper des Patienten implantiert ist.

22. Chirurgenset nach Anspruch 21, **dadurch gekennzeichnet, dass** das Messhilfsmittel (32) derart ausgeführt ist, dass es die Position des Probezwischenimplantats (106) in Bezug zum Probeschienbeinimplantat (104) bestimmt.

23. Chirurgenset nach Anspruch 22, **dadurch gekennzeichnet, dass** das Messhilfsmittel (32) ein erstes Tastelement (33), das eine erste Abtastfläche (33A) aufweist, und ein zweites Tastelement (34) umfasst, das eine zweite Abtastfläche (34A) aufweist, die dazu bestimmt sind, mit dem Probeschienbeinimplantat (104) bzw. dem Probezwischenimplantat (106) in Kontakt zu kommen.

24. Chirurgenset nach Anspruch 23, **dadurch gekennzeichnet, dass** das erste und das zweite Tastelement (33, 34) mit Mitteln zur Verbindung mit dem Probeschienbeinimplantat (104) bzw. dem Probezwischenimplantat (106) versehen sind, um einerseits mit diesen letztgenannten vor der Implantation der Probeknöchelprothese (101) verbunden zu werden und andererseits mit diesen letztgenannten verbunden zu bleiben, während sich die Brobeknöchelprothese (101) in dem Patienten befindet.

25. Chirurgenset nach Anspruch 23 oder 24, **dadurch gekennzeichnet, dass** das erste und das zweite Tastelement von einer ersten bzw. einer zweiten Verlängerung gebildet sind, die derart ausgeführt sind, dass sie jeweils, wenn die Probeknöchelprothese (101) implantiert ist, ein Ende aufweisen, das über den Schnitt, der durchgeführt wurde, um auf den Knöchel des Patienten zuzugreifen, hinausragt.

26. Chirurgenset nach einem der Ansprüche 23 bis 25, **dadurch gekennzeichnet, dass** das erste und das zweite Tastelement (33, 34) durch ein umkehrbares oder abnehmbares Feststellmittel verbunden sind, so dass sie zueinander beweglich oder hingegen miteinander verbunden bleiben können.

27. Chirurgensetz nach einem der Ansprüche 21 bis 26, **dadurch gekennzeichnet, dass** das Messhilfsmittel (32) auch derart ausgeführt ist, dass es die Übertragung der Konfiguration der Probeknöchelprothese (101) auf die endgültige Knöchelprothese (1) ermöglicht, wobei das Messhilfsmittel (32) so eine Vorlage für den Zusammenbau der endgültigen Knöchelprothese (1) bildet.
